# EUROPEAN PATENT SPECIFICATION

(11) **EP 0 963 377 B1**
(45) Date of publication and mention of the grant of the patent: **04.03.2009**
(21) Application number: 96932967.1
(22) Date of filing: 30.08.1996
(51) Int. Cl.: C07K 14/52, C07K 14/54, C12N 15/19, C12N 15/24, C07K 16/24, A61K 38/20, A61K 39/395, A61K 48/00

(54) **INTERLEUKIN-19**
INTERLEUKIN-19.
INTERLEUKINE 19

(43) Date of publication of application: 15.12.1999
(60) Divisional application: 08019123.2
(73) Proprietor: HUMAN GENOME SCIENCES, INC., Rockville, MD 20850 (US)
(72) Inventor: ROSEN, Craig, A., Laytonsville, MD 20882 (US); KENNY, Joseph, J., San Diego, CA 92129 (US)
(74) Representative: Vossius & Partner
(86) International application number: PCT/US1996/014355
(87) International publication number: WO 1998/008870

(56) References cited:
- DATABASE NCBI [Online] 28 April 1993 (1993-04-28) MARIDOR,G. ET AL.: "Chicken casein kinase II alpha subunit mRNA, complete cds." Database accession no. M59456 XP002196499
- DATABASE NCBI [Online] 28 October 1995 (1995-10-28) SANJANWALA AND WAAL-MALEFYT: "Human interleukin 10 (IL10) gene, complete cds." Database accession no. U16720 XP002196500
- ISHIDA H ET AL: "CONTINUOUS ANTI-INTERLEUKIN 10 ANTIBODY ADMINISTRATION DEPLETES MICE OF LY-1 B CELLS BUT NOT CONVENTIONAL B CELLS" JOURNAL OF EXPERIMENTAL MEDICINE, vol. 175, no. 5, 1992, pages 1213-1220, XP001068759 ISSN: 0022-1007
- VIEIRA P ET AL: "ISOLATION AND EXPRESSION OF HUMAN CYTOKINE SYNTHESIS INHIBITORY FACTOR COMPLEMENTARY DNA CLONES HOMOLOGY TO EPSTEIN-BARR VIRUS OPEN READING FRAME BCRFI" PROCEEDINGS OF THE NATIONAL ACADEMY OF SCIENCES OF THE UNITED STATES, vol. 88, no. 4, 1991, pages 1172-1176, XP002196532 1991 ISSN: 0027-8424
- NATURE, 19 July 1984, Vol. 310, BAER et al., "DNA Sequence and Expression of the B95-8 Epstein-Barr Virus Genome", pages 207-211.
- BIOCHEMICAL AND BIOPHYSICAL RESEARCH COMMUNICATIONS, 30 April 1993, Vol. 192, No. 2, FENG et al., "Molecular Cloning of Rat Cytokine Synthesis Inhibitory Factor (IL-10) cDNA and Expression in Spleen and Macrophages", pages 452-458.
- SCIENCE, 08 June 1990, Vol. 248, MOORE et al., "Homology of Cytokine Synthesis Inhibitory Factor (IL-10) to the Epstein-Barr Virus Gene BCRFI", pages 1230-1234.
- GENE, 1994, Vol. 139, HASH et al., "Characterization of a cDNA Encoding Bovine Interleukin 10: Kinetics of Expression in Bovine Lymphocytes", pages 257-261.
- PROC. NATL. ACAD. SCI. U.S.A., February 1991, Vol. 88, VIEIRA et al., "Isolation and Expression of Human Cytokine Synthesis Inhibitory Factor cDNA Clones: Homology to Epstein-Barr Virus Open Reading Frame BCRFI", pages 1172-1176.

### SEQUENCE LISTING

(1) GENERAL INFORMATION:
   (i) APPLICANT: Human Genome Sciences, Inc.
      9410 Key West Avenue
      Rockville, MD 20850
      United States of America
      APPLICANTS/INVENTORS: Rosen, Craig A. Kenny, Joseph J.
   (ii) TITLE OF INVENTION: INTERLEUKIN-19
   (iii) NUMBER OF SEQUENCES: 11
   (iv) CORRESPONDENCE ADDRESS:
      (A) ADDRESSEE: STERNE, KESSLER, GOLDSTEIN & FOX, P.L.L.C.
      (B) STREET: 1100 NEW YORK AVENUE, SUITE 600
      (C) CITY: WASHINGTON
      (D) STATE: DC
      (E) COUNTRY: USA
      (F) ZIP: 20005-3934
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: To be assigned
      (B) FILING DATE: 30-AUG-1996
      (C) CLASSIFICATION:
   (viii) ATTORNEY/AGENT INFORMATION:
      (A) NAME: Goldstein, Jorge A.
      (B) REGISTRATION NUMBER: 29,021
      (C) REFERENCE/DOCKET NUMBER: 1488.041PCOO
   (ix) TELECOMMUNICATION INFORMATION:
      (A) TELEPHONE: 202-371-2600
      (B) TELEFAX: 202-371-2540
(2) INFORMATION FOR SEQ ID NO:1:
   (i) SEQUENCE CHARACTERISTICS:
      (A) LENGTH: 966 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 44..574
   (ix) FEATURE:
      (A) NAME/KEY: sig_peptide
      (B) LOCATION: 44..115
   (ix) FEATURE:
      (A) NAME/KEY: mat_peptide
      (B) LOCATION: 116..574
   (xi) SEQUENCE DESCRIPTION: SEQ ID NO:1:
   GGCACGAGCA CAAGGAGCAG CCCGCAAGCA CCAAGTGAGA GGC ATG AAG TTA CAG       55
   TGT GTT TCC CTT TGG CTC CTG GGT ACA ATA CTG ATA TTG TGC TCA GTA      103
   GAC AAC CAC GGT CTC AGG AGA TGT CTG ATT TCC ACA GAC ATG CAC CAT      151
   ATA GAA GAG AGT TTC CAA GAA ATC AAA AGA GCC ATC CAA GCT AAG GAC      199
   ACC TTC CCA AAT GTC ACT ATC CTG TCC ACA TTG GAG ACT CTG CAG ATC      247
   ATT AAG CCC TTA GAT GTG TGC TGC GTG ACC AAG AAC CTC CTG GCG TTC      295
   TAC GTG GAC AGG GTG TTC AAG GAT CAT CAG GAG CCA AAC CCC AAA ATC      343
   TTG AGA AAA ATC AGC AGC ATT GCC AAC TCT TTC CTC TAC ATG CAG AAA      391
   ACT CTG CGG CAA TGT CAG GAA CAG AGG CAG TGT CAC TGC AGG CAG GAA      439
   GCC ACC AAT GCC ACC AGA GTC ATC CAT GAC AAC TAT GAT CAG CTG GAG      487
   GTC CAC GCT GCT GCC ATT AAA TCC CTG GGA GAG CTC GAC GTC TTT CTA       535
   GCC TGG ATT AAT AAG AAT CAT GAA GTA ATG TCC TCA GCT TGATGACAAG        584
   GAACCTGTAT AGTGATCCAG GGATGAACAC CCCCTGTGCG GTTTACTGTG GGAGACAGCC     644
   CACCTTGAAG GGGAAGGAGA TGGGGAAGGC CCCTTGCAGC TGAAAGTCCC ACTGGCTGGC     704
   CTCAGGCTGT CTTATTCCGC TTGAAAATAG CCAAAAAGTC TACTGTGGTA TTTGTAATAA     764
   ACTCTATCTG CTGAAAGGGC CTGCAGGCCA TCCTGGGAGT AAAGGGCTGC CTTCCCATCT     824
   AATTTATTGT GAAGTCATAT AGTCCATGTC TGTGATGTGA GCCAAGTGAT ATCCTGTAGT     884
   ACACATTGTA CTGAGTGGTT TTTCTGAATA AATTCCATAT TTTACCTATG GAAAAAAAAA     944
   AAAAAAAAAA AAAAAAAAAA AA                                              966
(2) INFORMATION FOR SEQ ID NO:2:
   (i) SEQUENCE CHARACTERISTICS:
      (A) LENGTH: 177 amino acids
      (B) TYPE: amino acid
      (D) TOPOLOGY: linear
   (ii) MOLECULE TYPE: protein
   (xi) SEQUENCE DESCRIPTION: SEQ ID NO:2:
(2) INFORMATION FOR SEQ ID.NO:3:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
      ACCTGAGGTC TGATGGCAAA GTCCAAGAAT 30
(2) INFORMATION FOR SEQ ID NO:4:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
      GGCATGCCAT GGAGTTACAG TGTGTTTCCC 30
(2) INFORMATION FOR SEQ ID NO:5:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
      GGAAGATCTA GCTGAGGACA TTAC 24
(2) INFORMATION FOR SEQ ID NO:6:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
      GGCGGGATCC CGCCATCATG AAGTTACAGT GTGTTTCCC 39
(2) INFORMATION FOR SEQ ID NO:7:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
      CCCAAGCTTG GTACCTCATC AAGCTGAGGA CATTAC 36
(2) INFORMATION FOR SEQ ID NO:8:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
      GGCGGGATCC CGCCATGAAG TTACAGTGTG TTTCCC 36
(2) INFORMATION FOR SEQ ID NO:9:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
   CCCAAGCTTG GTACCTCATC AGAAAGCGTA GTCTGGGACG TCGTATGGGT AAGCTGAGGA 60
   CATTACTTCA TGATT 75
(2) INFORMATION FOR SEQ ID NO:10:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
      GGCGGGATCC CGCCATCATG AAGTTACAGT GTGTTTCCC 39
(2) INFORMATION FOR SEQ ID NO:11:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
      CCCAAGCTTG GTACCTCATC AAGCTGAGGA CATTAC 36

## Description

### Background of the Invention

### Field of the Invention

The present invention relates to a human interleukin-19 (IL-19) protein and to polynucleotides encoding this protein.

### Related Art

Interlcukin-10 (IL-1 0) is a pleiotropic cytokine that has been implicated as an important regulator of the functions of lymphoid and myeloid cells. IL-10 blocks activation of cytokine synthesis and several accessory functions of macrophages, thus acting as a patent suppressor of the effector functions of macrophages, T cells and NK cells. IL-10 has also been implicated in the regulation of differentiation of B cells, mast cells and thymocytes.

IL-10 was identifies independently in two different lines of experiments. One of these identified a B-cell-derived mediator which co-stimulated active thymocytes (Suda et al., Cell Immunol 129:228 (1990)). The other identification determined that IL-10 is involved in the cross-regulation between two often mutually exclusive effector arms of immunity carried out by T helper (CD4⁺) subpopulations. Th1 (involved in cell-mediated immune response) mild Th2 (involved in antibody-mediated immune responses). In this role, IL-10 is expressed by Th2 cells and functions to suppress cytokine production by Th1 cells, an activity termed cytokine synthesis inhibitory factor (CSIF) activity.

DNA clones encoding murine IL-10 (mlL-10) were isolated based on the expression of CSIF activity (Moore et al., Science 248:1230-34 (1990)). cDNA clones encoding human IL-10 (hIL-10) were subsequently identified by cross-hybridization with the mouse cDNA (Vieira et al., Proc. Natl. Acad. Sci. USA 88:1172-1176 (1991)). mIL-10 is expressed by mouse CD4⁺ Th2 cells, at least one CD8⁺ clone, B lymphomas, T cells, activated mast cell lines, activate macrophages, keratinocytes, and Ly-1 B (B-1) cells (Fiorentino, D.F. et al., J. Exp. Med. 170:2081 (1989); (Moore et al., Science 248:1230-34 (1990); 87-93 (1992); Lin et al., Ann. N.Y. Acad. Sci. 651 O'Garra et al., Int. Immunol. 2: 821-832 (1990); MacNeil et al., J. Immunol. 145: 4167-4173 (1990); Florentino et al., J. Immunol. 147:3815-3822 (1991); Hisatsure et al, Lymphokine Cytokine Res. 11:651-683 (1992)). hIL-10 is expressed by human CD4⁺T cells and Th0, Th1, and Th2 T cell clones, by CD8⁺ T cells and clones (Yssel *et al., J. lmmunol.*), monocytes/macrophages, keratinocyces, activated B cells, B lymphomas, and Burkitt lymphoma lines infected with a transforming EBV strain, but not with a non-transforming strain (Vieira, P. et al., Proc. Natl. Acad. Sci. USA 88:1172-76 (1991); de Waal-Malefyt R. et al., J. Exp. Med 174:1209-20 (1991);de Waal-Malefyt, R. et al., J. Exp. Med. 174:915-24 (1991); Salgame, P. et al., Science 254:279-82 (1991):Yamamura, M. et al., Science 254:277-79 (1991);Ralph, P. et al., J. Immunol. 148:808-14 (1992);Benjamin, D. et al., Blood 80: 1289-98 (1992)). In particular, Vieira et al, PNAS, 1991, 1172-76 describes the human cytokine synthesis inhibitory factor (CSIF) [interleukin-10 (IL--10)], cDNA clones encoding human IL-10 isolated from a tetanus toxin-specific human T-cell clone. hIL-10 is considered to exhibit strong DNA and amino acid sequence homology to an open reading frame in the Epstein-Barr virus, BCRFI and hIL-10 and the BCRFI product inhibit cytokine synthesis by activated human peripheral blood mononuclear cells and by a mouse Th1 clone. Thus, IL-10 is not strictly a Th2-specific cytokine, and its pattern of expression resembles IL-6 more than IL-4 or IL-5 (Wang, S. C. et al., Transplant. Proc. 23:2920-22 (1991)). Like IL-6 but unlike most other T cell derived cytokines, IL-10 expression is not inhibited by cyclosporin or FK-506 (Wang, S.C. el al., Transplant Proc. 23:2920 (1991)).

In an attempt to determine the *in vivo* role of IL-10, normal mice were treated from birth to adulthood with IL-10 - neutralizing antibodies (Wang, S.C. et al., Transplant Proc. 23:2920 (1991); Ishida, H. et al., J. Exp. Med. 175:1213 (1992)) The resulting phenotypic changes included an increased level of circulating IFN-γ, TNF-α and IL-6, reduced serum IgM and IgA, a marked deletion of peritoneal B cells, and an inability to develop *in vivo* antibody responses to two bacterial antigens known be combatted with antibody produced by peritoneal B cells (Hayakawa, K. el al., Annu. Rev. Immunol. 6:197 (1988)). The reduction in peritoneal B cells was determined to be a consequence of IFN-γ elevation (Ishida, H. et al., J. Exp. Med. 175:1213 (1992)).

Other experiments have shown that IL-10 suppresses *in vitro* production of inflammatory monokines such as TNF-α and IL-1. This data corresponds to *in vivo* studies which show that IL-10 antagonists elevate the same inflammatory monokines. These results predict a strong anti-inflammatory role for IL-10. In addition, IL-10 antagonists may be useful to enhance Th1 immunity, which could be beneficial in infectious diseases of viral origin, or diseases involving intracellular pathogens.

The diverse biological activities of IL-10 have led to predictions that both IL-10 and its antagonists .will have a wide range of clinical applications. It is clear that there is a continuing need in the art for isolating novel cytokines capable of mediating such diverse biological processes.

### Summary of the Invention

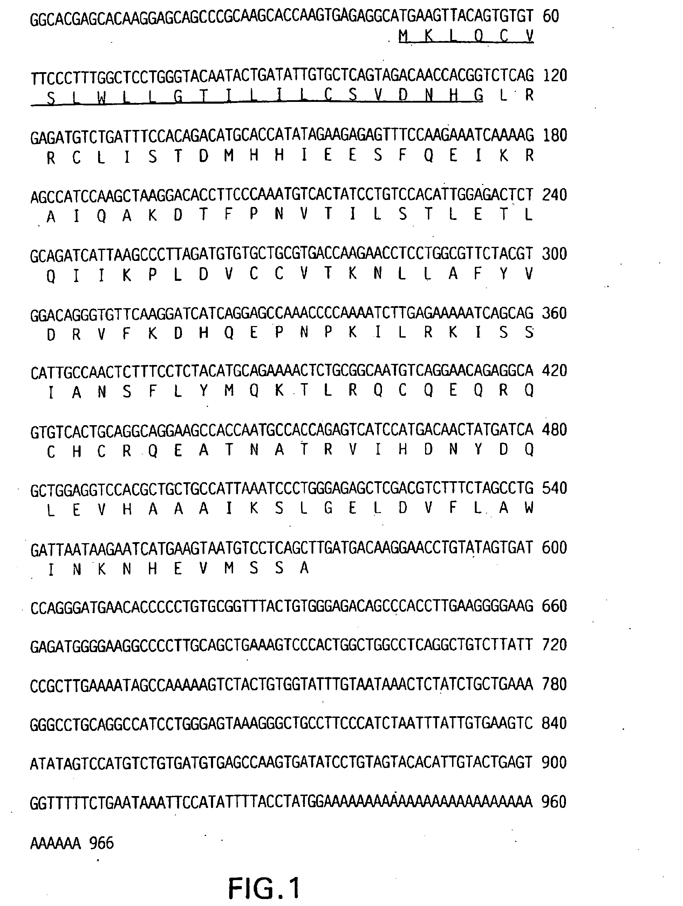

The present invention provides isolated nucleic acid molecules comprising a polynucleotide encoding a human IL-19 polypeptide having the amino acid sequence in Figure 1 [SEQ ID NO: 2] or the amino acid sequence encoded by the cDNA clone deposited as ATCC Deposit Number 97662 on July 17, 1996. The nucleotide sequence determined by sequencing the deposited IL-19, cDNA clone, which is shown in Figure 1 [SEQ ID NO: 1], contains an open reading frame encoding a polypeptide of about I77 amino acid residues including an initiation codon at nucleotide positions 44-46, a leader sequence of about 24 amino acid residues and a deduced molecular weight of about 20.4 kDa. The 153 amino acid sequence of the predicted mature IL-19 protein is shown in Figure 1 (last 153 residues) and in SEQ ID NO:2 (from amino acid residue 1 to residue 153).

Thus, one aspect of the invention provides isolated nucleic acid molecules comprising a polynucleotide having a nucleotide sequence selected from the group consisting of: (a) a nucleotide sequence encoding the IL-19 polypeptide having the complete amino acid sequence shown in Figure 1 [SEQ ID NO:2]; (b) a nucleotide sequence encoding the mature IL-19 polypeptide having the amino acid sequence at positions 25-177 in Figure 1 [residues 1-153 of SEQ ID NO:2]; (c) a nucleotide sequence encoding the IL-19 polypeptide having the complete amino acid sequence encoded by the cDNA clone contained in ATCC Deposit No. 97662: (d) a nucleotide sequence encoding the mature IL-19 polypeptide having the amino acid sequence encoded by the cDN4 clone contained in ATCC Deposit No. 97662; and (e) a nucleotide sequence complementary to any or the nucleotide sequences in (a), (b), (c) or (d) above. Preferably, the nucleic acid molecule will encode the mature polypeptide as shown in Figure 1 [SEQ ID NO: 2] or as encoded by the above-described deposited cDNA.

Further embodiments of the invention include isolated nucleic acid molecules that comprise a polynucleotide having a nucleotide sequence at least 90% identical, and more preferably at least 95%, 97% 98% or 99% identical, to any of the nucleotide sequences in (a), (b), (c), (d) or (e) above, and which encodes a polypeptide having IL-19 activity, wherein said activity is the property of modulating the synthesis of at least one cytokine in the group consisting of IFN-γ, lymphotoxin, IL-2, IL-3 and GM-CSF in a population of T-helper cells induced to synthesize one or more of these cytokines by exposure to syngeneic antigen presenting cells (APC) and antigen, and/or the complementary strand of said polynucleotide hybridizes under stringent hybridization conditions to a polynucleotide having a nucleotide sequence identical to a nucleotide sequence in (a), (b), (c), (d) or (e), above. The polynucleotide which hybridizes does not hybridize under stringent hybridization conditions to a polynucleotide having a nucleotide sequence consisting of only A resides or of only T residues. An additional nucleic acid embodiment of the invention relates to an isolated nucleic acid molecule comprising a polynucleotide which encodes the amino acid sequence of an epitope-bearing portion of an IL-19 polypeptide having an amino acid sequence in (a), (b), (c) or (d), above.

The present invention also relates to recombinant vectors which include the isolated nucleic acid molecules of the present invention and to host cells containing the recombinant vectors, as well as to methods of making such vectors and host cells and for using them for production of IL-19 polypeptides or peptides by recombinant techniques.

The invention further provides an isolated IL-19 polypeptide having amino acid sequence selected from the group consisting of: (a) the amino acid Sequence of the IL-19 polypeptide having the complete 177 amino acid sequence including the leader sequence shown in Figure 1 [SEQ ID NO:2]; (b) the amino acid sequence of the mature IL-19 polypeptide (without the leader) having the amino acid sequence at positions 25-177 in Figure 1 [residues 1-153 of SEQ ID NO:2] (c) the amino acid sequence of the 1L-19 polypeptide having the complete amino acid sequence including the leader encoded by the cDNA clone contained in ATCC Deposit neo. 97662; and (d) th- amino acid sequence of the mature IL-19 polypeptide having the amino acid sequence encoded by the cDNA clone contained in ATCC Deposit No. 97662. The polypeptides of the present invention also include polypeptides having an amino acid sequence with at least 90% similarity, more preferably ai least 95% similarity to those described in (a), (b), (c) or (d) above, well as polypeptides having an amino acid sequence at least 80% identical, more preferably at least 90% identical, and still more preferably 95%, 97%, 98% or 99% identical to those above.

An additional embodiments of this aspect of the invention relates to a peptide or polypeptide which has the amino acid sequence of an epitope-bearing portion of an IL-19 polypeptide having an amino acid sequence described in (a), (b), (c) or epitope-bearing portion of an IL-19 polypeptide of the invention include portions of such polypeptides with at least six or seven, preferably at least nine, and more preferably at least about 30 amino acids to about 50 amino acids, although epitope-bearing polypeptides of any length up to and including the entire amino acid sequence of a polypeptide of the invention described above also are included in the invention, wherein said immunogenic epitope is a portion of the polypeptide that elicits an antibody response when the whole polypeptide is the specifically to an IL-19 polypeptide having an amino acid sequence described in (a), (b), (c) or (d) above.

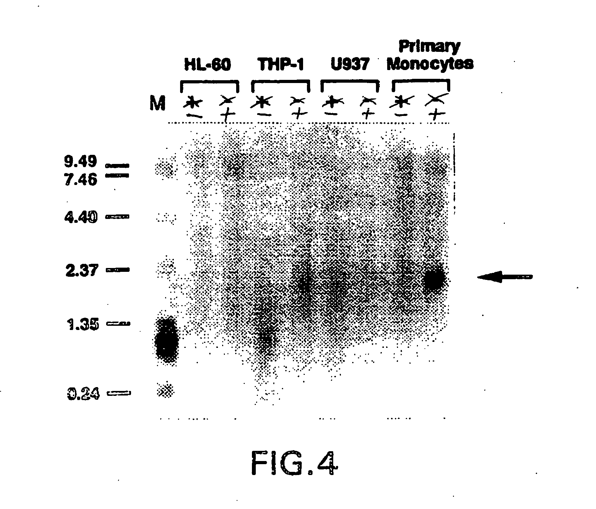

IL-19, which is secreted, and which has significant homology to IL-10, is believed by the present inventors to be expressed only, or at least primarily, in activated monocytes (Figure 4). Thus, detecting IL-19 gene expression in cells of the immune system is useful for identifying activated monocytes. Further, for a number of disorders, it is believed by the inventors that significantly higher or lower levels of IL-19 gene expression can be detected in bodily fluids (e.g., serum, plasma, urine, synovial fluid or spinal fluid) taken from an individual having such a disorder, relative to a "standard" IL-19 gene expression level, i.e., the IL-19 expression level in bodily fluids from an individual not having the disorder. Thus, the application describes a diagnostic method useful during diagnosis of a disorder related to an abnormal level of IL-19 gene expression, which involves (a) assaying IL-19 gene expression level in cells or body fluid of that individual; (b) comparing that IL-19 gene expression level with a standard IL-19 gene expression level, whereby an increase or decrease in the assayed IL-19 gene expression level compared to the standard expression level is indicative of a disorder. An additional aspect of the invention is related to a use for the preparation of a pharmaceutical composition for treating an individual in need of decreased levels of IFN-γ, TNF-α and/or IL-6 which involves administering to such an individual a composition comprising and IL-19 polypeptide of the invention.

### Brief Description of the Figures

Figure 1 shows the nucleotide [SEQ ID NO: 1] and deduced amino acid [SEQ ID NO:2] sequences of the complete IL-19 protein determined by sequencing of the DNA clone contained in ATCC Deposit No. 97662. The protein has a leader sequence of about 24 amino acid residues (underlined) and a deduced molecular weight of about 20.4 kDa. The amino acid sequence of the predicted mature IL-19 protein is also shown in Figure 1 (last 153 amino acids) [residues 1-153 of SEQ ID NO: 2].
Figure 2 is a protein gel showing IL-19 protein expressed from E. coli strain M15rep4 (see Example 1).
Figure 3 is a protein gel showing full length and truncated IL-19 proteins produced in an *in vitro* coupled transcription/translation system (see Example 3).
Figure 4 is a northern blot analysis of IL-19 expression in human tissue (HL-60, THP-1, U937 and primary human monocytes) (see Example 5).
Figure 5 shows the regions of similarity between the amino acid sequences of the IL-19 protein and human IL-10 (hIL-10) [SEQ ID NO: 3].

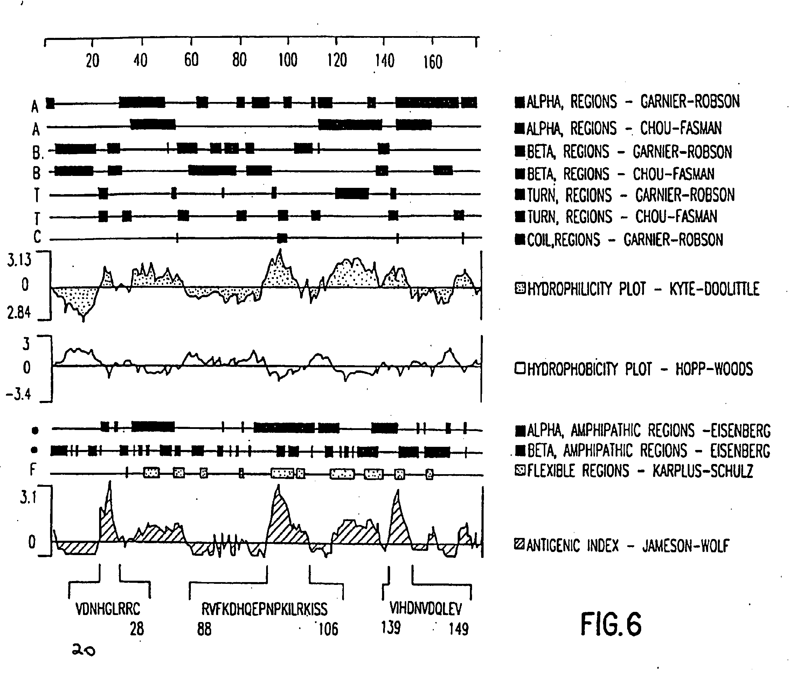

Figure 6 shows an analysis of the IL-19 amino acid sequence, as generated by the Protean module of the DNA* Star sequence analysis computer software package. The alpha helix, beta sheet, and turn regions predicted by Chou-Fasman, Garnier-Robson, and Eisenberg methods are shown. The hydrophilicity and hydrophobicity profiles of IL-19 predicted by the Kyte-Doolittle and Hopp-Woods methods, respectively, are also shown. The antigenic index of the IL-19 amino acid sequence predicted by the Jameson-Wolf method is also shown. The amino acid residues of IL-19 which correspond to the peak regions (amino acids 20-28, 88-106, and 139-149) of the Jameson-Wolf antigenic index are shown below the antigenic index plot.

### Detailed Description of the Invention

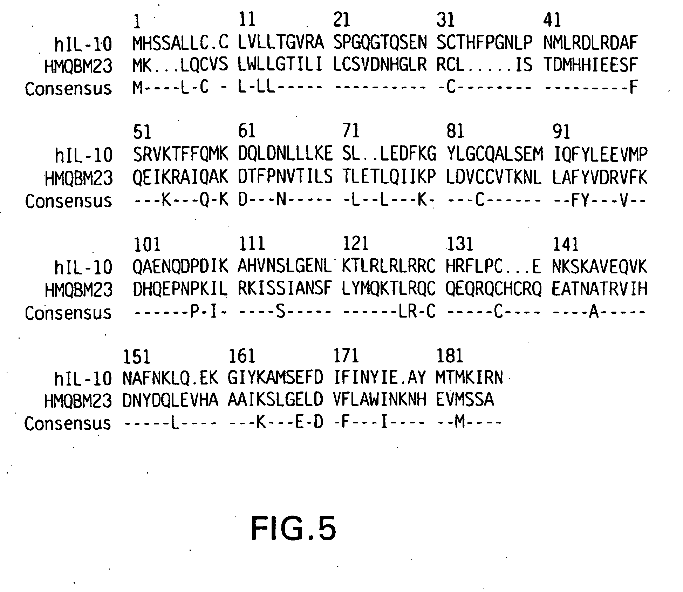

The present invention provides isolated nucleic acid molecules comprising a polynucleotide encoding the IL-19 protein having the amino acid sequence shown in Figure 1 [SEQ ID NO:2] which was determined by sequencing a cloned cDNA: The IL-19 protein of the present invention shares sequence homology with human IL-10 (Figure 5) [SEQ ID NO:3]. The nucleotide sequence shown in Figure 1 [SEQ ID NO:1] was obtained by sequencing the HMQBM23 cDNA clone encoding an IL-19 polypeptide, which was deposited on July 17, 1996 at the American Type Culture Collection, 12301 Park Lawn Drive, Rockville, Maryland 20852, and given accession number 97662. The deposited clone is contained in the pBluescript SK(-) plasmid (Stratagene, La Jolla, CA).

### Nucleic Acid Molecules

Unless otherwise indicated, all nucleotide sequences determined by sequencing a DNA molecule herein were determined using an automated DNA sequencer (such as the Model 373 from Applied Biosystems, Inc.), and all amino acid sequences of polypeptides encoded by DNA molecules determined herein were predicted by translation of a DNA sequence determined as above. Therefore, as is known in the art for any DNA sequence determined by this automated approach, any nucleotide sequence determined herein may contain a some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

Unless otherwise indicated, each "nucleotide sequence" set forth herein is presented as a sequence of deoxyribonucleotides (abbreviated A, G , C and T). However, by "nucleotide sequence" of a nucleic acid molecule or polynucleotide is intended, for a DNA molecule or polynucleotide, a sequence of deoxyribonucleotides, and for an RNA molecule or polynucleotide, the corresponding sequence of ribonucleotides (A, G, C and U) where each thymidine deoxynucleotide (T) in the specified deoxynucleotide sequence in is replaced by the ribonucleotide uridine (U). For instance, reference to an RNA molecule having the sequence of SEQ ID NO:1 set forth using deoxyribonucleotide abbreviations is intended to indicate an RNA molecule having a sequence in which each deoxynucleotide A, G or C of SEQ ID NO:1 has been replaced by the corresponding ribonucleotide A, G or C, and each deoxynucleotide T has been replaced by a ribonucleotide U.

Using the information provided herein, such as the nucleotide sequence in Figure 1, a nucleic acid molecule of the present invention encoding an IL-19 polypeptide may be obtained using standard cloning and screening procedures, such as those for cloning cDNAs using mRNA as starting material. Illustrative of the invention, the nucleic acid molecule described in Figure 1 [SEQ ID NO: 1] was discovered in a cDNA library derived from human activated monocytes. The determined nucleotide sequence of the IL-19 cDNA of Figure 1 contains an open reading frame encoding a protein of about 177 amino acid residues with an initiation codon at positions 44-46 of the nucleotide sequence shown in Figure 1 [SEQ ID NO. 11, and a predicted leader sequence of about 24 amino acid residues, and a deduced molecular weight of about 20.4 kDa. The amino acid sequence of the predicted mature IL-19 protein is also shown in Figure 1 [residues-1-153 of SEQ ID NO:2] from about amino acid residue 25 to about residue 177. The IL-19 protein shown in Figure 1 SEQ ID NO:2] is about 20% identical and about 46% similar to human IL-10 (Figure 5).

As one of ordinary skill would appreciate, due to the possibilities of sequencing errors discussed above, as well as the variability of cleavage sites for leaders in different known proteins, the actual IL-19 polypeptide encoded by the deposited cDNA comprises about 177 amino acids, but may be anywhere in the range of 170-183 amino acids; and the actual leader sequence of this protein is about 24 amino acids, but may be anywhere in the range of about 18 to about 29 amino acids.

As indicated, nucleic acid molecules of the present invention may be in the form of RNA, such as mRNA, or in the form of DNA, including, for instance, cDNA and genomic DNA obtained by cloning or produced synthetically. The DNA may be double-stranded or single-stranded. Single-stranded DNA or RNA may be the coding strand, also known as the sense strand, or it may be the non-coding strand, also referred to as the anti-sense strand.

By "isolated" nucleic acid molecule(s) is intended a nucleic acid molecule, DNA or RNA, which has been removed from its native environment For example, recombinant DNA molecules contained in a vector are considered isolated for the purposes of the present invention. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the DNA molecules of the present invention. Isolated nucleic acid molecules according to the present invention further include such molecules produced synthetically.

Isolated nucleic acid molecules of the present invention include DNA molecules comprising an open reading frame (ORF) with an initiation codon at positions 44-46 of the nucleotide sequence shown in Figure 1 [SEQ ID NO:1]; DNA molecules comprising the coding sequence for the mature IL-19 protein shown in Figure 1 (last 153 amino acids) [residues 1-153 of SEQ ID NO:2]; and DNA molecules which comprise a sequence substantially different from those described above but which, due to the degeneracy of the genetic code, still encode the IL-19 protein. Of course, the genetic code is well known in the art. Thus, it would be routine for one skilled in the art to generate the degenerate variants described above.

In another aspect, the invention provides isolated nucleic acid molecules encoding the IL-19 polypeptide having an amino acid sequence encoded by the cDNA clone contained in the plasmid deposited as ATCC Deposit No. 97662 on July 17, 1996. Preferably, this nucleic acid molecule will encode the mature polypeptide encoded by the above-described deposited cDNA clone. The invention further provides an isolated nucleic acid molecule having the nucleotide sequence shown in Figure 1 [SEQ ID NO: 1] or the nucleotide sequence of the IL-19 cDNA contained in the above-described deposited clone, or nucleic acid molecule having a sequence complementary to one of the above sequences. Such isolated molecules, particularly RNA molecules, are useful as probes for gene mapping by in situ hybridization with chromosomes and for detecting expression of the IL-19 gene in human tissue, for instance, by Northern blot analysis

In another aspect, the invention provides an isolated nucleic acid molecule comprising a polynucleotide which hybridizes under stringent hybridization conditions to a portion of the polynucleotide in a nucleic acid molecule of the invention described above, for instance, the cDNA clone contained in ATCC Deposit No. 97662. By "stringent hybridization conditions" is intended overnight incubation at 42 °C in a solution comprising: 50% formamide, 5x SSC (750 mM NaCl, 75mM trisodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C. By a polynucleotide which hybridizes to a "portion" of a polynucleotide is intended a polynucleotide (either DNA or RNA) hybridizing to at least about 15 nucleotides (nt), and more preferably at least about 20 nt, still more preferably at least about 30 nt, and even more preferably about 30-70 nt of the reference polynucleotide. These are useful as diagnostic probes and primers as discussed above and in more detail below.

Of course, polynucleotides hybridizing to a larger portion of the reference polynucleotide (e.g., the deposited cDNA clone), for instance, a portion 50-750 nt in length, or even to the entire length of the reference polynucleotide, also useful as probes according to the present invention, as are polynucleotides corresponding to most, if not all, of the nucleotide sequence of the deposited cDNA or the nucleotide sequence as shown in Figure 1 [SEQ ID NO:1]. By a portion of a polynucleotide of "at least 20 nt in length," for example, is intended 20 or more contiguous nucleotides from the nucleotide sequence of the reference polynucleotide, (e.g., the deposited cDNA or the nucleotide sequence as shown in Figure 1 [SEQ ID NO:1]). As indicated, such portions are useful diagnostically either as a probe according to conventional DNA hybridization techniques or as primers for amplification of a target sequence by the polymerase chain reaction (PCR), as described, for instance, in Molecular Cloning, A Laboratory Manual, 2nd. edition, edited by Sambrook, J., Fritsch, E. F. and Maniatis, T., (1989), Cold Spring Harbor Laboratory Press.

Since an IL-19 cDNA clone has been deposited and its determined nucleotide sequence is provided in Figure 1 [SEQ ID NO:1], generating polynucleotides which hybridize to a portion of the IL-19 cDNA molecule would be routine to the skilled artisan. For examples, restriction endonuclease cleavage or shearing by sonication of the IL-19 cDNA clone could easily be used to generate DNA portions of various sizes which are polynucleotides that hybridize to a portion of the IL-19 cDNA molecule. Alternatively, the hybridizing polynucleotides of the present invention could be generated synthetically according to known techniques. Of course, a polynucleotide which hybridizes only to a poly A sequence (such as the 3' terminal poly(A) tract of the IL-19 cDNA shown in Figure 1 [SEQ ID NO:1]), or to a complementary stretch of T (or U) resides, would not be included in a polynucleotide of the invention used to hybridize to a portion of a nucleic acid of the invention, since such a polynucleotide would hybridize to any nucleic acid molecule contain a poly (A) stretch or the complement thereof (e.g., practically any double-stranded DNA clone.

The present application further describes fragments of the isolated nucleic acid molecules described herein. By a fragment of an isolated nucleic acid molecule having the nucleotide sequence of the deposited cDNA or the nucleotide sequence shown in Figure 1 (SEQ ID NO:1) is intended fragments at least about 15 nt, and more preferably at least about 20 nt, still more preferably at least about 30 nt, and even more preferably, at least about 40 nt in length which are useful as diagnostic probes and primers as discussed herein Of course, larger fragments 50-950 nt in length are also useful as are fragments corresponding to most, if not all, of the nucleotide sequence of the deposited cDNA or as shown in Figure 1 (SEQ ID NO: 1). By a fragment at least 20 nt in length, for example, is intended fragments which include 20 or more contiguous bases from the nucleotide sequence of the deposited cDNA or the nucleotide sequence as shown in Figure 1 (SEQ ID NO:1). Since the gene has been deposited and the nucleotide sequence shown in Figure 1 (SEQ ID NO:1) is provided, generating such DNA fragments would be routine to the skilled artisan. For example, restriction endonuclease cleavage or shearing by sonication could easily be used to generate fragments of various sizes. Alternatively, such fragments could be generated synthetically.

Preferred nucleic acid fragments of the present invention include nucleic acid molecules encoding epitope-bearing portions of the IL-19 protein. In particular, such nucleic acid fragments of the present invention include nucleic acid molecules encoding: a polypeptide comprising amino acid residues from about 20 to about 28 in Figure 1 (residues -5 to 4 of SEQ ID NO:2); a polypeptide comprising amino acid residues from about 88 to about 106 in Figure 1 (residues 64-82 of SEQ ID NO:2); and a polypeptide comprising amino acid residues from about 139 to about 149 in Figure 1 (residues 115-125 of SEQ ID NO:2). The inventors have determined that the above polypeptide fragments are antigenic regions of the IL-19 protein. Methods for determining other such epitope-bearing portions of the IL-19 protein are described in detail below.

The invention further provides isolated nucleic acid molecules comprising a polynucleotide encoding an portion of the IL-19 protein. In particular, isolated nucleic acid molecules are provided encoding polypeptides comprising the following amino acid residues in Figure 1 (SEQ ID NO:2), which the present inventors have determined are antigenic regions of the IL-19 protein: residues 20-28, residues 88-106, and residues 139-149 (see Figure 6). Methods for generating such epitope bearing portions of IL-19 are described in detail below.

As indicated, nucleic acid molecules of the present invention which encode the IL-19 polypeptide may include, but are not limited to those encoding the amino acid sequence of the mature polypeptide, by itself; the coding sequence for the mature polypeptide and additional sequences, such as those encoding the about 24 amino acid leader or secretory sequence, such as a pre-, or pro- or prepro- protein sequence; the coding sequence of the mature polypeptide, with or without the aforementioned additional coding sequences, together with additional, non-coding sequences, including for example, but not limited to introns and non-coding 5' and 3' sequences, such as the transcribed, non-translated sequences that play a role in transcription, mRNA processing - including splicing and polyadenylation signals, for example ribosome binding and stability of mRNA; an additional coding sequences which codes for additional amino acids, such as those which provide additional functionalities. Thus, the sequence encoding the polypeptide may be fused to a marker sequence, such as a sequence encoding a peptide which facilitates purification of the fused polypeptide. In certain preferred embodiments of this aspect of the invention, the marker amino acid sequence is a hexa-histidine peptide, such as the tag provided in a pQE vector (Qiagcn, Inc.), among others, many of which are commercially available. As described in Gentz et al. (1989) Proc. Natl. Acad. Sci., USA 86:821-824, for instance, hexa-histidine provides for convenient purification of the fusion protein. The "HA" tag is another peptide useful for purification, which corresponds to an cpitope derived from the influenza hemagglutinin protein, which has been described by Wilson et al., Cell 37: 767 (1984).

The present application further describes variants of the nucleic acid molecules of the present invention, which encode portions, analogs or derivatives of the IL-19 protein. Variants may occur naturally, such as a natural allelic variant. By an "allelic variant" is intended one of several alternate forms of a gene occupying a given locus on a chromosome of an organism. *Genes II,* Lewin, ed. Non-naturally occurring variants may be produced using art-known mutagenesis techniques.

Such variants include those produced by nucleotide substitutions, deletions or additions. The substitutions, deletions or additions may involve one or more nucleotides. The variants may be altered in coding or non-coding regions or both. Alterations in the coding regions may produce conservative or non-conservative amino acid substitutions, deletions or additions. Especially preferred among these are silent substitutions, additions and deletions, which do not alter the properties and activities of the IL-19 protein or portions thereof. Also especially preferred in this regard are conservative substitutions. Most highly preferred are nucleic acid molecules encoding the mature IL-19 protein having the amino acid sequence shown in Figure 1 [SEQ ID NO:2] or the mature IL-19 amino acid sequence encoded by the deposited cDNA clone.

Further embodiments of the invention include isolated nucleic acid molecules comprising a polynucleotide having a nucleotide sequence at least 90% identical, and more preferably at least 95%, 97%, 98% or 99% identical to (a) a nucleotide sequence encoding the full-length IL-19 polypeptide having the complete amino acid sequence in Figure 1 [SEQ ID NO:2] including the predicted leader sequence; (b) a nucleotide sequence encoding the mature IL-19 polypeptide (full-length polypeptide with the leader removed) having the amino acid sequence at positions 25-177 in Figure 1 [residues 1-153 of SEQ ID NO:2]; (c) a nucleotide sequence encoding the full-length IL-19 polypeptide having the complete amino acid sequence including the leader encoded by the cDNA clone contained in ATCC Deposit No. 97662; (d) a nucleotide sequence encoding the mature IL-19 polypeptide having the amino acid sequence encoded by the cDNA clone contained in ATCC Deposit No. 97662; or (e) a nucleotide sequence complementary to any of the nucleotide sequences in (a), (b), (c) or (d).

By a polynucleotide having a nucleotide sequence at least, for example, 95% "identical" to a reference nucleotide sequence encoding an IL-19 polypeptide is intended that the nucleotide sequence of the polynucleotide is identical to the reference sequence except that the polynucleotide sequence may include up to five point mutations per each 100 nucleotides of the reference nucleotide sequence encoding the IL-19 polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

As a practical matter, whether any particular nucleic acid molecule is at least 90%, 95%, 97%, 98% or 99% identical to, for instance, the nucleotide sequence shown in Figure 1 or to the nucleotides sequence of the deposited cDNA clone can be determined conventionally using known computer programs such as the Bestfit program (Wisconsin Sequence Analysis Package, Version 8 for Unix, Genetics Computer Group, University Research Park, 575 Science Drive, Madison, WI 53711. Bestfit uses the local homology algorithm of Smith and Waterman (Advances in Applied Mathematics 2: 482-489, 1981) to find the best segment of homology between two sequences. When using Bestfit or any other sequence alignment program to determine whether a particular sequence is, for instance, 95% identical to a reference sequence according to the present invention, the parameters are set, of course, such that the percentage of identity is calculated over the full length of the reference nucleotide sequence and that gaps in homology of up to 5% of the total number of nucleotides in the reference sequence are allowed.

The present application is directed to nucleic acid molecules at least 90%, 95%, 97%, 98% or 99% identical to the nucleic acid sequence shown in Figure 1 [SEQ ID NO:1] or to the nucleic acid sequence of the deposited cDNA, irrespective of whether they encode a polypeptide having IL-19 activity. This is because, even where a particular nucleic acid molecule does not encode a polypeptide having IL-19 activity, one of skill in the art would still know how to use the nucleic acid molecule, for instance, as a hybridization probe or a polymerase chain reaction (PCR) primer. Uses of the nucleic acid molecules of the present invention that do not encode a polypeptide having IL-19 activity include, *inter alia,* (1) isolating the IL-19 gene or allelic variants thereof in a cDNA library; (2) *in situ* hybridization (e.g., "FISH") to metaphase chromosomal spreads to provide precise chromosomal location of the IL-19 gene as described in Verma et al., Human Chromosomes: a Manual of Basic Techniques, Pergamon Press, New York (1988); and Northern Blot analysis for detecting IL-19 mRNA expression in specific cell types (e.g., activated monocytes).

Preferred, however, are nucleic acid molecules having sequences at least 90%, 95%, 97%, 98% or 99% identical to the nucleic acid sequence shown in Figure 1 [SEQ ID NO:1] or to the nucleic acid sequence of the deposited cDNA which do, in fact, encode a polypeptide having IL-19 protein activity. By "a polypeptide having IL-19 activity" is intended polypeptides exhibiting activity similar, but not necessarily identical, to an activity of the IL-19 protein of the invention (either the full-length protein or, preferably, the mature protein) as measured in a particular biological assay.

IL-19 exhibits several biological activities which could form the basis of such biological assays. In particular, it is believed by the inventors that IL-19 has the property of modulating the synthesis of at least one cytokine in the group consisting of IFN-γ, lymphotoxin, IL-2, IL-3, and GM-CSF in a population of T helper cells induced to synthesize one or more of these cytokines by exposure to syngeneic antigen-presenting cells (APCs) and antigen. In this activity, APCs are treated so that they become incapable of replication, but their antigen-processing machinery remains functional. This is conveniently accomplished by irradiating the APCs, e.g. with about 1500-3000 R (gamma or X-radiation) before mixing with the T-cells.

Alternatively, changes in levels of cytokine production may be assayed in primary or, preferably, secondary mixed-lymphocyte reactions (MLR), in which case syngeneic APCs need not be used. MLRs are well-known in the art, e.g., Bradley, pp 162-166 in Mishell et al., eds. Selected Methods in Cellular Immunology (Freeman, San Francisco, 1980); and Battisto, et al., Meth, in Enzymol. 150:83-91 (1987). Briefly, the cell populations are mixed, one of the populations having been treated prior to mixing to prevent proliferation, e.g., by irradiation. Preferably, the cell populations are prepared at a concentration of about 2 X 10⁶ cells/ml in supplemented media, e.g. RPMI 1640 with 10% fetal calf serum. For both controls and test cultures, mix .05ml of each population for the assay. For a secondary MLR, the cells remaining after 7 days in the primary MLR are re-stimulated by freshly prepared, irradiated stimulator cells. The sample suspected of containing IL-19 may be added to the test cultures at the time of mixing, and both controls and test cultures may be assayed for cytokine production from 1-3 days after mixing.

Obtaining T cell populations and/or APC populations for IL-19 assays employs techniques well known in the art which are fully described in DiSabato et al., eds., Meth in Enzymol. Vol. 108 (1984). APCs for the preferred IL-19 assays are peripheral blood monocytes. These are obtained using standard techniques, e.g. as described by the following articles in the aforementioned DiSabato et a;., eds., Meth. in Enzymol. Vol. 108 (1984): Boyum, pp. 88-102; Mage, pp. 118-132; Litvin et al., pp. 298-302; Stevenson, pp. 242-249; and Romain, pp. 148-153.

Preferably, helper T-cells are used in the IL-19 assays, which are obtained by first separating lymphocytes from the peripheral blood, and then selecting, e.g., by paining or flow cytometry, helper cells using a commercially available anti-CD4 antibody, e.g. OKT4, described in U.S. Patent No. 4,381,295, and available form Ortho Pharmaceutical Corp. The requisite technique are fully disclosed by Boyum in Scand. JL. Clin. Lab. Invest., 21(Suppl. 97):77 (1968) and in Meth. in Enzymol. Vol. 108 (1984) *(cited above)* and by Bram et al., Meth. in Enzymol. 121:737-748 (1986). Generally, PBLs are obtained from fresh blood by Ficoll-Hypaque density gradient centrifugation.

A variety of antigens can be employed in the assay, e.g. Keyhole limpet hemocyanin (KLH), fowl γ-globulin, or the like. More preferably, in place of antigen, helper T cells are stimulated with anti-CD3 monoclonal antibody, e.g. OKT3 disclosed in U.S. Patent No. 4,361,5449, in the assay.

Cytokine concentrations in control and test samples are measured by standard biological and/or immunochemical assays. Construction of immunochemical assays for specific cytokines is well known in the art when the purified cytokine is available; e.g. Campbell, Monoclonal Antibody Technology (Elsevier, Amsterdam, 1984); Tijssen, Practice and Theory of Enzyme Immunoassays (Elsevier, Amsterdam, 1985); and U.S. Patent No. 4,486,530, are examples of the extensive literature on the subject. ELISA kits for human IL-2, human IL-3, and human GM-CSF are commercially available from Genzyme Corp. (Boston, MA); and an ELISA kit for human IFN-γ is commercially available from Endogen. Inc. (Boston, MA). Polyclonal antibodies specific for human lymphotoxin are available from enzyme, Corp., which can be used in a radio immunoassay for human lymphotoxin, e.g., Chard, An Introduction to Radioimmunoassay and Related Techniques (Elsevier, Amsterdam, 1982).

Biological assays of the cytokines listed above can also be used to determine whether a sample has IL-19 activity, i.e, whether a sample modulates cytokine expression or activity in a manner similar to IL-19. A biological assay for human lymphotoxins disclosed by Aggarwal, Meth in Enzymol. 116:441-447 (1985), and by Matthews et al., in Lymphokines and Interferons: A Practical Approach, Clemens et al., eds, IRL Press, Washington, D.C., 1987, pp. 221-225. Human IL-2 and GM-CSF can be assayed with factor-dependent cell lines CTLL-2 and KG-1, available from the ATCC under accession numbers TIB 214 and CCL246, respectively. Human IL-3 can be assayed by its ability to simulate the formation of a wide range of hematopoietic cell colonies in soft agar cultures, e.g. as described by MEtcalf, The Hematopoietic Colony Stimulating Factors (Elsevier, Amsterdam, 19844). IFN-γ can be quantified with anti-viral assays, e.g. Meager, pp. 129-147, in Clemens et al., eds (cited above).

Cytokine production can also be determined by mRNA analysis. Cytokine mRNAs can be measured by cytoplasmic dot hybridization, as described by White et al. (J. Biol. Chem. 257:8569-8572 (1982)) and Gillespie et al., U.S. Patent No. 4,483,920. Other approaches include dot blotting using purified RNA, e.g. Chapter 6 in Hames et al., eds, Nucleic Acid Hybridization: A Practical Approach, IRL PRess, Washington D.C., 1985.

Some samples to be tested for IL-19 activity must be pretreated to remove cytokines that might interfere with the assays For example, IL-2 increases the production of IFN-γ in some cells. Thus, depending on the T helper cells used in the assay, IL-2 may have to be removed from the sample being tested. Such removals are conveniently accommodated by passing a sample over a standard anti-cytokine affinity column.

Thus, by using an assay such as those described above, the effect of the substance suspected of having IL-19 activity on the activity of any one of a number of cytokines may be compared to the IL-19 protein of the invention, in order to determine if the sample indeed has IL-19 activity.

Of course, due to the degeneracy of the genetic code, one of ordinary skill in the art will immediately recognize that a large number of the nucleic acid molecules having a sequence at least 90%, 95%, 97%, 98%, or 99% identical to the nucleic acid sequence of the deposited cDNA or the nucleic acid sequence shown in Figure 1 [SEQ ID NO:1] will encode a polypeptide "having IL-19 protein activity." In fact; since degenerate variants of these nucleotide sequences all encode the same polypeptide, this will be clear to the skilled artisan even without performing one of the above-described comparison assays. It will be further recognized in the art that, for such nucleic acid molecules that are not degenerate variants, a reasonable numbers will also encode a polypeptide having IL-19 protein activity. This is because the skilled artisan is fully aware of amino acid substitutions that are either less likely or not likely to significantly effect protein function (e.g., replacing one aliphatic amino acid with a second aliphatic amino acid).

For examples, guidance concerning how to make phenotypically silent amino acid substitutions is provided in Bowie, J. U., et al., "Deciphering the Message in Protein Sequences: Tolerance to Amino Acid Substitutions," Science 247:1306-1310 (1990), wherein the authors indicate that there are two main approaches for studying the tolerance of an amino acid sequence to change. The first method relies on the process of evolution, in which mutations are either accepted or rejected by natural selection. The second approach uses genetic engineering to introduce amino acid changes at specific positions of a cloned gene and selections or screens to identify sequences that maintain functionality. As the authors state, these studies have revealed that proteins are surprisingly tolerant of amino acid substitutions. The authors further indicate which amino acid changes are likely to be permissive at a certain position of the protein. For example, most buried amino acid residues require nonpolar side chains, whereas few features of surface side chains are generally conserved. Other such phenotypically silent substitutions are described in Bowie, J.U., *et al., supra,* and the references cited therein.

### Vectors and Host Cells

The present invention also relates to vectors which include the isolated DNA molecules of the present invention, host cells which are genetically engineered with the recombinant vectors, and the production of IL-19 polypeptides or portions thereof by recombinant techniques.

Recombinant constructs may be introduced into host cells using well known techniques such as infection, transduction, transfection, transvection, electroporation and transformation. The vector may be, for example, a phage, plasmid, viral or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged *in vitro* using an appropriate packaging cell line and then transduced into host cells.

Preferred are vectors comprising cis-acting control regions to the polynucleotide of interest. Appropriate trans-acting factors may be supplied by the host, supplied by a complementing vector or supplied by the vector itself upon introduction into the host.

In certain preferred embodiments in this regard, the vectors provide for specific expression, which may be inducible and/or cell type-specific. Particularly preferred among such vectors are those inducible by environmental factors that are easy to manipulate, such as temperature and nutrient additives.

Expression vectors useful in the present invention include chromosomal-, episomal- and virus-derived vectors, e:g., vectors derived from bacterial plasmids, bacteriophage, yeast episomes, yeast chromosomal elements, viruses such as baculoviruses, papova viruses, vaccinia viruses, adenoviruses, fowl pox viruses, pseudorabies viruses and retroviruses, and vectors derived from combinations thereof, such as cosmids and phagemids.

The DNA insert should be operatively linked to an appropriate promoter, such as the phage lambda PL promoter, the *E. coli lac, trp* and *lac* promoters, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will include a translation initiating AUG at the beginning and a termination codon appropriately positioned at the end of the polypeptide to be translated.

As indicated, the expression vectors will preferably include at least one selectable marker. Such markers include dihydrofolate reductase or neomycin resistance for eukaryotic cell culture and tetracycline or ampicillin resistance genes for culturing in *E. coli* and other bacteria. Representative examples of appropriate hosts include bacterial cells, such as *E. coli,* Streptomyces and *Salmonella typhimurium* cells; fungal cells, such as yeast cells; insect cells such as Drosophila S2 and Spodoptera Sf9 cells; animal cells such as CHO, COS and Bowes melanoma cells; and plant cells. Appropriate culture media and conditions for the above-described host cells are known in the art.

Among vectors preferred for use in bacteria include pA2, pQE70, pQE60 and pQE-9, available from Qiagen; pBS vectors, Phagescript vectors, Bluescript vectors, pNH8A, pNH1a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Among known bacterial promoters suitable for use in the present invention include the *E. coli lac*I and *lac*Z promoters, the T3 and T7 promoters, the *gpt* promoter, the lambda PR and PL promoters and the *trp* promoter. Suitable eukaryotic promoters include the CMV immediate early promoter, the HSV thymidine kinase promoter, the early and late SV40 promoters, the promoters of retroviral LTRs, such as those of the Rous sarcoma virus (RSV), and metallothionein promoters, such as the mouse metallothionein-I promoter.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis et al., BASIC METHODS IN MOLECULAR BIOLOGY, (1986).

Transcription of the DNA encoding the polypeptides of the present invention by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

For secretion of the translated protein into the lumen of the endoplasmic reticulum, into the periplasmic space or into the extracellular environment, appropriate secretion signals may be incorporated into the expressed polypeptide. The signals may be endogenous to the polypeptide or they may be heterologous signals.

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-O 464 533 (Canadian counterpart 2045869) discloses fusion proteins comprising various portions of constant region of immunoglobin molecules together with another human protein or part thereof. In many cases, the Fc part in a fusion protein is thoroughly advantageous for use in therapy and diagnosis and thus results, for example, in improved pharmacokinetic properties (EP-A 0232 262). On the other hand, for some uses it would be desirable to be able to delete the Fc part after the fusion protein has been expressed, detected and purified in the advantageous manner described. This is the case when Fc portion proves to be a hindrance to use in therapy and diagnosis, for example when the fusion protein is to be used as antigen for immunizations. In drug discovery, for example, human proteins, such as, hIL5 has been fused with Fc portions for the purpose of high-throughput screening assays to identify antagonists of hIL-5. See, D. Bennett et al., Journal of Molecular Recognition, Vol. 8 52-58 (1995) and K. Johanson et al., The Journal of Biological Chemistry, Vol. 270, No. 16, pp 9459-9471 (1995).

The IL-19 protein can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

### IL-19 Polypeptides and Peptides

The invention further provides an isolated IL-19 polypeptide having the amino acid sequence encoded by the deposited cDNA, or the amino acid sequence in Figure 1 [SEQ ID NO:2], or a peptide or polypeptide comprising a portion of the above polypeptides. The terms "peptide" and "oligopeptide" are considered synonymous (as is commonly recognized) and each term can be used interchangeably as the context requires to indicate a chain of at least to amino acids coupled by peptidyl linkages. The word "polypeptide" is used herein for chains containing more than ten amino acid residues. All oligopeptide and polypeptide formulas or sequences herein are written from left to right and in the direction from amino terminals to carboxy terminus.

It will be recognized in the art that some amino acid sequence of the IL-19 polypeptide can be varied without significant effect of the structure or function of the protein. If such differences in sequence are contemplated, it should be remembered that there will be critical areas on the protein which determine activity. In general, it is possible to replace residues which form the tertiary structure, provided that residues performing a similar function are used. In other instances, the type of residue may be completely unimportant if the alteration occurs at a non-critical region of the protein.

Thus, the application further describes variations of the IL-19 polypeptide which show substantial IL-19 polypeptide activity or which included regions of IL-19 protein such as the protein portions discussed below. Such mutants include, deletions, insertions, inversions, repeats, and type substitutions (for example, substituting one hydrophilic residue for another, but not strongly hydrophilic for strongly hydrophobic as a rule). Small changes or such "neutral" amino acid substitutions will generally have little effect on activity.

Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacement among the aromatic residues Phe, Tyr.

As indicated in detail shove, further guidance concerning which amino acid changes are likely to be phenotypically silent (i.e., are not likely to have a significant deleterious effect on a function) can be found in Bowie, J.U., et al., "deciphering the Message in Protein Sequences: Tolerance to Amino Acid Substitutions," Science 247:1306-1310 (1990).

The polypeptides of the present invention are preferably provided in an isolated form, and preferably are substantially purified. A recombinantly produced version of the IL-19 polypeptide can be substantially purified by the one-step method described in Smith and Johnson, Gene 67: 31-40 (1988).

The polypeptides of the present invention include the polypeptide encoded by the deposited cDNA including the leader, the mature polypeptide encoded by the deposited the cDNA minus the leader (i.e., the mature protein), the polypeptide of Figure 1 [SEQ ID NO:2] including the leader, the polypeptide of Figure 1 [SEQ ID NO:2] minus the leader, as well as polypeptides which have at least 90% similarity, more preferably at least 95% similarity, and still more preferably at least 97%, 98% or 99% similarity to those described above. Further polypeptides of the present invention include polypeptides at least 80% identical, more preferably at least 90% or 95% identical, still ; more preferably at least 97%, 98% or 99% identical to the polypeptide encoded by the deposited cDNA, to the polypeptide of SEQ ID NO:2, and also include portions of such polypeptides with at least 30 amino acids and more preferably at least 50 amino acids, wherein said polypeptide has IL-19 activity, wherein said activity is the property of modulating the synthesis of at least one cytokine in the group consisting of IFN-γ, lymphotoxin, IL-2, IL-3 and GM-CSF in a population of T-helper cells induced to synthesize one or more of these cytokines by exposure to syngeneic antigen presenting cells (APC) and antigen.

By "% similarity" for two polypeptides is intended a similarity score produced by comparing the amino acid sequences of the two polypeptides using the Bestfit program (Wisconsin Sequence Analysis Package, Version 8 for Unix, Genetics Computer Group, University research Park, 575 Science Drive. Madison, WI 53711) and the default settings for determining similarity. Bestfit uses the local homology algorithm of Smith and Waterman (Advances in Applied Mathematics 2: 482-489, 1981) to find the best segment of similarity between two sequences.

By a polypeptide having an amino acid sequence at least, for example, 95% "identical" to a reference amino acid sequence of an IL-19 polypeptide is intended that the amino acid sequence of the polypeptide is identical to the reference sequence except that the polypeptide sequence may include up to five amino acid alterations per each 100 amino acids of the reference amino acid of the IL-19 polypeptide. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 97%, 98% or 99% identical to, for instance, the amino acid sequence shown in Figure 1 [SEQ ID NO:2] or to the amino acid sequence encoded by deposited cDNA clone can be determined conventionally using known computer programs such the Bestfit program (Wisconsin Sequence Analysis Package, Version 8 for Unix, Genetics Computer Group, University Research Park, 575 Science Drive, Madison, WI 53711. When using Bestfit or any other sequence alignment program to determine whether a particular sequence is, for instance, 95% identical to a reference sequence according to the present invention, the parameters are set, of course, such that the percentage of identity is calculated over the full length of the reference amino acid sequence and that gaps in homology of up to 5% of the total number of amino acid residues in the reference sequence are allowed.

As described in detail below, the polypeptides of the present invention can be used to raise polyclonal and monoclonal antibodies, which are useful in diagnostic assays for detecting I-19 protein expression as described below or as agonists and antagonists capable of enhancing or inhibiting IL-19 protein function. Further, such polypeptides can be used in the yeast two-hybrid system to "capture" IL-19 protein binding proteins which are also candidate agonist and antagonist according to the present invention. The yeast two hybrid system is described in Fields and Song, Nature 340:245-246 (1989).

In another aspect, the invention provides a peptide or polypeptide comprising an epitope-bearing portion of a polypeptide of the invention. The epitope of this polypeptide portion is an immunogenic-epitope- of a polypeptide of the invention. An "immunogenic epitope" is defined as a part of a protein that elicits an antibody response when the whole protein is the immunogen. These immunogenic epitope are believed to be confined to a few loci on the molecule. On the other hand, a region of a protein molecule to which an antibody can bind is defined as an "antigenic epitope." The number of "immunogenic epitopes of a protein generally is less than the number of antigenic epitopes. See, for instance, Geysen, H. M., Meloen, R. H. and Barteling, S. J. (1984) Use of peptide synthesis to probe viral antigens for epitopes to a resolution of a single amino acid. Proc. Natl. Acad. Sci. USA 81:3998-4002.

As to the selection of peptides or polypeptides bearing art antigenic epitope (i.e., that contain a region of a protein molecule to which a antibody can bind), it is well known in that art that relatively short synthetic peptides that mimic part of a protein sequence are routinely capable of eliciting an antiserum that reacts with the partially mimicked protein. See, for instance, Sutcliffe, J. G., Shinnick, T. M., Green, N. and Learner, R. A. (1983) Antibodies that react with predetermined sites on proteins. Science 219:660-666. Peptides capable of eliciting protein-reactive sera are frequently represented in the primary sequence of a protein, can be characterized by a set of simple chemical rules, and are confined neither to immunodominant regions of intact protein, (i.e., immunogenic epitopes) nor to the amino or carboxyl terminals. Peptides that are extremely hydrophobic and those of six or fewer residues generally are ineffective at inducing antibodies that bind to the mimicked protein; longer, soluble peptides, especially those containing proline residues, usually are effective. Sutcliffe et al., *supra*, at 661. For instance, 18 of 20 peptides designed according to these guidelines, containing 8-39 residues covering 75% of the sequence of the influenza virus hemagglutinin HAl polypeptide chain, induced antibodies that reacted with the HAI protein or intact virus; and 12/12 peptides from the MuLV polymerase and 18/18 from the rabies glycoprotein induced antibodies that precipitated the respective proteins.

Antigenic epitope-bearing peptidesand polypeptides as described in the application are therefore useful to raise antibodies, including monoclonal antibodies, that bind specifically to a polypeptide of the invention. Thus, a high proportion of hybridomas obtained by fusion of spleen cells from donors immunized with an antigen epitope-bearing peptide generally secrete antibody reactive with the native protein. Sutcliffe et al., *supra,* at 663. The antibodies raised by antigenic epitope-bearing peptides or polypeptides are useful to detect the mimicked protein, and antibodies to different peptides may be used for tracking the fate of various regions of a protein precursor which undergoes posttranslation processing. The peptides and anti-peptide antibodies may be used in a variety of qualitative or quantitative assays for the mimicked protein, for instance in competition assays since it has been shown that even short peptides (e.g., about 9 amino acids) can bind and displace the larger peptides in immunoprecipitation assays. See, for instance, Wilson, 1. A., Niman, H. L., Houghten, R. A., Cherenson, A. R., Connolly, M. L. and Lerner, R. A. (1984) The structure of an antigenic determinant in a protein. Cell 37:767-778 at 777. The anti-peptide antibodies of the invention also are useful for purification of the mimicked protein, for instance, by adsorption chromatography using methods well known in the art.

Antigenic epitope-bearing peptides described in the application and polypeptides of the invention designed according to the above guidelines preferably contain a sequence of at least seven, more preferably at least nine and most preferably between about 15 to about 30 amino acids contained within the amino acid sequence of a polypeptide of the invention. However, peptides or polypeptides comprising a larger portion of an amino acid sequence of a polypeptide of the invention, containing about 30 to about 50 amino acids, or any length up to and including the entire amino acid sequence of a polypeptide, of the invention, also are considered epitope-bearing peptides or polypeptides of the invention and also are useful for inducing antibodies that react with the mimicked protein. Preferably, the amino acid sequence of the epitope-bearing peptide is selected to provide substantial solubility in aqueous solvents (i.e., the sequence includes relatively hydrophilic residues and highly hydrophobic sequences are preferably avoided); and sequences containing proline residues are particularly preferred.

Non-limiting examples of antigenic polypeptides or peptides that can be used to generate IL-19-specific antibodies include: a polypeptide comprising amino acid residues from about 20 to about 28 in Figure 1 (residues -5 to 4 of SEQ ID NO:2); a polypeptide comprising amino acid residues from about 88 to about106 in Figure 1 (residues 64-82 of SEQ ID NO:2); and a polypeptide comprising amino acid residues from about 139 to about 149 in Figure 1 (residues 115-125 of SEQ ID NO:2). As indicated above, the inventors have determined that the above polypeptide fragments are antigenic regions of the endokine alpha protein.

The epitope-bearing peptides and polypeptides of the invention may be produced by any conventional means for making peptides or polypeptides including recombinant means using nucleic acid molecules of the invention. For instance, a short epitope-bearing amino acid sequence may be fused to a larger polypeptide which acts as a carrier during recombinant production and purification, as well as during immunization to produce anti-peptide antibodies. Epitope-bearing peptides also may be synthesized using known methods of chemical synthesis. For instance, Houghten has described a simple method for synthesis of large numbers of peptides, such as 10-20 mg of 248 different 13 residue peptides representing single amino acid variants of a segment of the HAI 1 polypeptide which were prepared and characterized (by ELISA-type binding studies) in less than four weeks. Houghten, R. A. (1985) General method for the rapid solid-phase synthesis of large numbers of peptides: specificity of antigen-antibody interaction at the level of individual amino acids. Proc. Natl. Acad. Sci. USA 82:5131-5135. This "Simultaneous Multiple Peptide Synthesis (SMPS)" process is further described in U.S. Patent No. 4,631,211 to Houghten et aI. (1986). In this procedure the individual resins for the solid-phase synthesis of various peptides are contained in separate solvent-permeable packets, enabling the optimal use of the many identical repetitive steps involved in solid-phase methods. A completely manual procedure allows 500-1000 or more syntheses to be conducted simultaneously. Houghten et al., *supra,* at 5134.

Epitope-bearing peptides and polypeptides of the invention are used to induce antibodies according to methods well known in the art. See, for instance, Sutcliffe et al., *supra;* Wilson et al., *supra;* Chow, M., Yabrov, R., Bittle, J., Hogel, J. and Baltimore, D., Proc. Natl. Acad. Sci. USA 82:910-914; and Bittle, F. J., Fry, C. M., Rowlands, D. J., Brown, F., Bittle, J. L., Houghten, R. A. and Lerner, R. A. (1985) J. gen. Virol. 66:2347-2354. Generally, animals may be immunized with free peptide; however, anti-peptide antibody titer may be boosted by coupling of the peptide to a macromolecular carrier, such as keyhole limpet hemacyanin (KLH) or tetanus toxoid. For instance, peptides containing cysteine may be coupled to carrier using a linker such as m-maleimidobenzoyl-N-hydroxysuccinimide ester (MBS), while other peptides may be coupled to carrier using a more general linking agent such as glutaraldehyde. Animals such as rabbits, rats and mice are immunized with either free or carrier-coupled peptides, for instance, by intraperitoneal and/or intradermal injection of emulsions containing about 100 µg peptide or carrier protein and Freund's adjuvant. Several booster injections may be needed, for instance, at intervals of about two weeks, to provide a useful titer of anti-peptide antibody which can be detected, for example, by ELISA assay using free peptide adsorbed to a solid surface. The titer of anti-peptide antibodies in serum from an immunized animal may be increased by selection of anti-peptide antibodies, for instance, by adsorption to the peptide on a solid support and elution of the selected antibodies according to methods well known in the art.

Immunogenic epitope-bearing peptides of the invention, i.e., those parts of a protein that elicit an antibody response when the whole protein is the immunogen, are identified according to methods known in the art. For instance, Geysen et al., 1984, *supra,* discloses a procedure for rapid concurrent synthesis on solid supports of hundreds of peptides of sufficient purity to react in an enzyme-linked immunosorbent assay. Interaction of synthesized peptides with antibodies is then easily detected without removing them from the support. In this manner a peptide bearing an immunogenic epitope of a desired protein may be identified routinely by one of ordinary skill in the art. For instance, the immunologically important epitope in the coat protein of foot-and-mouth disease virus was located by Geysen et al. with a resolution of seven amino acids by synthesis of an overlapping set of all 208 possible hexapeptides covering the entire 213 amino acid sequence of the protein. Then, a complete replacement set of peptides in which all 20 amino acids were substituted in turn at every position within the epitope were synthesized, and the particular amino acids conferring specificity for the reaction with antibody were determined. Thus, peptide analogs of the epitope-bearing peptides of the invention can be made routinely by this method. U.S. Patent No. 4,708,781 to Geysen (1987) further describes this method of identifying a peptide bearing an immunogenic epitope of a desired protein.

Further still, U.S. Patent No. 5,194,392 to Geysen (1990) describes a general method of detecting or determining the sequence of monomers (amino acids or other compounds) which is a topological equivalent of the epitope (i.e., a "mimotope") which is complementary to a particular paratope (antigen binding site) of an antibody of interest. More generally, U.S. Patent No. 4,433,092 to Geysen (1989) describes a method of detecting or determining a sequence of monomers which is a topographical equivalent of a ligand' which is complementary to the ligand binding site of a particular receptor of interest. Similarly, U.S. Patent No. 5,480,971 to Houghten, R A. et al. (1996) on Peralkylated Oligopeptide Mixtures discloses linear C₁-C₇-alkyl peralkylated oligopeptides and sets and libraries of such peptides, as well as methods for using such oligopeptide sets and libraries for determining the sequence of a peralkylated oligopeptide that preferentially binds to an acceptor molecule of interest. Thus, non-peptide analogs of the epitope-bearing peptides of the invention also can be made routinely by these methods.

As one of skill in the an will appreciate, IL-19 polypeptides of the present invention and the epitope-bearing fragments thereof described above can be combined with parts of the constant domain of immunoglobulins (IgG), resulting in chimeric polypeptides. These fusion proteins facilitate purification and show an increased half-life *in vivo.* This has been shown, e.g., for chimeric proteins consisting of the first two domains of the human CD4-polypeptide and various domains of the constant regions of the heavy or light chains of mammalian immunoglobulins (EPA 394,827; Traunecker et al., Nature 331:84- 86 (1988)). Fusion proteins that have a disulfide-linked dimeric structure due to the IgG part can also be more efficient in binding and neutralizing other molecules than the monomeric IL-19 protein or protein fragment alone (Fountoutakis et, al., J Biochem 270:3958-3964 (1995)).

### Chromosome Assays

The nucleic acid molecules of the present invention are also valuable for chromosome identification. The sequence is specifically targeted to and can hybridize with a particular location on an individual human chromosome. Moreover, there is a current need for identifying particular sites on the chromosome. Few chromosome marking reagents based en actual sequence data (repeat polymorphisms) are presently available for marking chromosomal location. The mapping of DNAs to chromosome according to the present invention is an important first step in correlating those sequences with genes associated with disease.

In certain preferred embodiments in this regard, the DNA herein disclosed is used to clone genomic DNA of an interleukin-19 protein gene. This can be accomplished using a variety of well known techniques and libraries, which generally are available commercially. The genomic DNA then is used for *in situ* chromosome mapping using well known techniques for this purpose. Typically, in accordance with routine procedures for chromosome mapping, some trial and error may be necessary to identify a genomic probe that gives a good *in situ* hybridization signal.

In some cases, in addition, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified portion.

PCR mapping of somatic cell hybrids is a rapid procedure for assigning a particular DNA to a particular chromosome. Using the present invention with the same oligonucleotide primers, sublocalization can be achieved with panels of portions from specific chromosomes or pools of large genomic clones in an analogous manner. Other mapping strategies that can similarly be used to map to its chromosome include in situ hybridization, prescreening with labeled flow-sorted chromosomes and preselection by hybridization to construct chromosome specific-cDNA libraries.

Fluorescence *in situ* hybridization ("FISH") of a cDNA clone to a metaphase chromosomal spread can be used to provide a precise chromosomal location in one step. This technique can be used with probes from the cDNA as short as 50 or 60 bp. For a review of this technique, see Verma et al., HUMAN CHROMOSOMES: A MANUAL OF BASIC TECHNIQUES, Pergamon Press, New York (1988).

Once a sequence has been mapped to a precise chromosomal location, the physical position of the sequence on the chromosome can be correlated with genetic map data. Such data are found, for example, in V. McKusick, MENDELIAN INHERITANCE IN MAN, available on-line through Johns Hopkins University, Welch Medical Library. The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

### Treatment of Pathological Conditions by IL-19 Inhibition of Cytokine Production

As noted above, IL-19 is secreted by activated monocytes, and shares significant homology with human IL-10. Thus, it is believed by the inventors that I-19 is active in inhibiting cytokine production during the mammalian immune response. The cytokines whose production may be affected by IL-19 include IFN-γ, TNF-α, and IL-6.

One such activity of IL-19 is the ability to limit excessive production of gamma-interferon (IFN-γ), and hence the consequent effects of such production, including major histocompatibility (MHC) associated auto-immune diseases. As increased IFN-γ expression has been implicated in an increase of MHC genes, which may then increase the chance of an autoimmune response against the MHC-overexpressing cells, the ability to limit IFN-γ expression may be therapeutically valuable in the treatment of clinical manifestations of such MHC disorders. These include rheumatoid arthritis, systemic lupus erythematosus (SLE), myasthenia gravis, insulin-dependent diabetes mellilitus, and thyroiditis.

The down regulation of IFN-γ by IL-19 may also be therapeutically valuable in treating parasitic infections such as leishmaniasis. Levels of IFN-γ, IL-2 and IL-4 are all involved in the regulation of the life cycle of this parasite. Thus, the ability to regulate the production of these cytokines by IL-19 will be therapeutically valuable.

Given the activities modulated by IL-19, it is readily apparent that a substantially altered (increased or decreased) level of expression of IL-19 in an individual compared to the standard or "normal" level produces pathological conditions such as those described above. It will also be appreciated by one of ordinary skill that, since the IL-19 protein of the invention is translated with a leader peptide suitable for secretion of the mature protein from the cells which express IL-19, when IL-19 protein (particularly the mature form) is added from an erogenous source to cells, tissues or the body of an individuals, the protein will exert its modulating activities on any of its target cells. Therefore, it will be appreciated that conditions caused by a decrease in the standard or normal level of IL-19 activity in an individual, can be treated be administration of IL-19 protein. Thus, the application further describes a method of treating and individual in need of an increased level of IL-19 activity comprising administering to such an individual a pharmaceutical composition comprising an amount of an isolated IL-19 polypeptide of the invention, particularly a mature form of the IL-19 protein of the invention, effective to increase the IL-19 activity level in such an individual.

One of ordinary skill will appreciate that effective amounts of the IL-19 polypeptides for treating an individual in need of an increased level of IL-19 activity can be determined empirically for each condition where administration of IL-19 is indicated. The polypeptide having IL-19 activity my be administered in pharmaceutical compositions in combination with one or more pharmaceutically acceptable excipients. It will be underwood that, when administered to a human patient, the total daily usage of the pharmaceutical compositions of the present invention will be decided by the attending physician within the scope of sound medical judgment. The specific therapeutically effective dose level for any particular patient will depend upon a variety of factors including the type and degree of the response to be achieved; the specific composition an other agent, if any, employed; the age, body weight, general health, sex and diet of the patient; the time of administration, route of administration, and rate of excretion of the composition; the duration of the treatment; drugs (such as a chemotherapeutic agent) used in combination or coincidental with the specific composition; and like factors well known in the medical arts.

For example, it is predicted that satisfactory results are obtained by oral administration of a polypeptide having IL-19 activity in dosages on the order of from 0.05 to 10 mg/kg/day, preferably 0.1 to 7.5 mg/kg/day, more preferably 0.1 to 2 mg/kg/day, administered once or, in divided doses, 2 to 4 times per day. On administration parenterally, for example by i.v. drip or infusion, dosages on the order of from 0.01 to 5 mg/kg/day, preferably 0.05 to 1.0 mg/kg/day and more preferably 0.1 to 1.0 mg/kg/day can be used. Suitable daily dosages for patients are thus on the order of from 2.5 to 500 mg p.o., preferably 5 to 250 mg p.o., more preferably 5 to 100 mg p.o., or on the order of from 0.5 to 250 mg i.v., preferably 2.5 to 125 mg i.v. and more preferably 2.5 to 50 mg i.v.

Dosaging may also be arranged in a patient specific manner to provide a predetermined concentration of an IL-19 activity in the blood, as determined by an RIA technique, for instance. Thus patient dosaging may be adjusted to achieve regular on-going trough blood levels, as measured by RIA, on the order of from 50 to 1000 ng/ml, preferably 150 to 500 ng/ml.

Pharmaceutical compositions of the invention may be administered orally, rectally, parenterally, intracistemally, intravaginally, intraperitoneally, topically (as by powders, ointments, drops or transdermal patch), bucally, or as an oral or nasal spray. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

Pharmaceutical compositions of the present invention for parenteral injection can comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

The compositions of the present invention may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption such as aluminum monostearate and gelatin.

In some cases, in order to prolong the effect of the pharmaceutical composition, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsulated matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active compounds are mixed with at least one item pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f)-absorption accelerators such as quaternary ammonium compounds, g) wetting agents such as, for example, cetyl alcohol and glycerol monostearate, h)-absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isoptopyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

The active polypeptide can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to the agent or inhibitor, stabilizers, preservatives, excipients, and the like. The preferred lipids are the phospholipids and the phosphatidyl cholates (lecithins), both natural and synthetic. Methods to form liposomes are known in the art. See, for example, Prescott, Ed., Methods in Cell Biology, Volume XIV, Academic Press, New York, N.Y. (1976), p. 33 et seq*.*

### Use of IL-19 in Adoptive Immunotherapy of Cancer

Another therapeutic application of IL-19 is the administration of IL-19 in adoptive immunotherapy to prevent or reduce the production of cytokines believed to be responsible for many of the deleterious side effects currently encountered in adoptive immunotherapy. As used herein, "Adoptive immunotherapy" means therapy wherein functional cancer-fighting immune cells are transferred to a patient. The cancer-fighting immune cells preferably comprise tumor infiltrating lymphocytes (TILs) originating from the patient himself. As is known in the art, while IL-2 is useful in adoptive immunotherapy due to its ability to activate the killer cells transferred to the patent (Rosenberg et al., Ann. Rev. Immuno/. 4: 681-709 (1988)), the severe side effects caused directly or indirectly by IL-2 have been an obstacle to the development of routine treatment protocols based on this approach (Hsu, D-H et al., WO 92/12726). As IL-19 is believed to be capable of preventing or reducing the production of cytokines responsible for these side effects, TILs cultured in the presence of both IL-2 and IL-19 prior to administration, wherein the administration of IL-2 and IL-19 is continued after the administration of those TILs to the patient, may reduce the deleterious side effects typical of adoptive immunotherapy.

### Use of IL-19 Antagonists in the Restoration of Immunocompetency to T Helper Cells in HIV-Infected Patients

Another therapeutic application of the IL-19 polypeptide of the invention is the use of the polypeptide to identify IL-19 antagonists, such as an antibody specific for binding to IL-19, which can then be used to increase the production of IL-2 in T helper cells. For example, patients infected with human immunodeficiency virus (HIV) have a decreased level of IL-2 production in non-virally infected T helper cells. IL-19 is believed to be capable of preventing or reducing the production of IL-2. Therefore, the administration of IL-19 antagonists may result in an increase in IL-2 production in HIV-infected patients. As IL-2 is responsible for T cell proliferation, the maintenance of IL-2 production is beneficial to HIV-infected patients.

Antagonists specific for IL-19 can be made by mutating the amino acid sequence of IL-19 using standard mutagenesis methods well known to those of ordinary skill in the art. Such methods include the use of M13 vectors to introduce single-site mutations, to delete random amino acids from IL-19, or to add amino acids. The resulting muteins are then tested in standard assays for the ability to compete with non-mutated IL-19, including assays which test the ability of the mutein, as compared with the IL-19 protein of the invention, to enhance IL-2 dependent proliferation of T cells *in vitro.*

Other suitable IL-19 antagonists include an antibody specific for binding to IL-19 (αIL-19) which interferes with its binding to the T helper receptor. Production of such antibodies has been described in full above.

The antibodies used in the method of the invention preferably are autologous for the patient, thereby minimizing further immunological problems. However, as immunodeficient individuals in need of this treatment will tend to be less reactive to non-self antibodies, non-self antibodies derived from cells of the same species will also be useful.

Having generally described the invention, the same will be more readily understood by reference to the following examples, which are provided by way of illustration and are not intended as limiting.

### Examples

### Example 1: Expression and Purification of IL-19 in E. coli

The DNA sequence encoding the mature IL-19 protein in the deposited cDNA clone was amplified using PCR oligonucleotide primers specific to the amino terminal sequences of the IL-19 protein and to vector sequences 3' to the gene. Additional nucleotides containing restriction sites to facilitate cloning were added to the 5' and 3' sequences respectively.

The 5' oligonucleotide primer had the sequence 5' GGC ATG CCA TGG AGT TAC AGT GTG TTT CCC 3' [SEQ IQ NO:4] (sequences specific to the IL-19 nucleotide sequence are underlined), and included an *Nco*I restriction site.

The 3' primer had the sequence 5' GGA AGA TCT AGC TGA GGA CAT TAC 3' [SEQ ID NO:5] containing the underlined 15 nucleotides complementary to the last 15 nucleotides immediately after the IL-19 protein coding sequence in Figure 1. The 3' primer included a *Bgl*II restriction site.

The restriction sites were convenient to restriction enzyme sites in the bacterial expression vector pQE60, which were used for bacterial expression in these examples. (Qiagen, Inc. 9259 Eton Avenue, Chatsworth, CA, 91311). pQE60 encodes ampicillin antibiotic resistance ("Amp"') and contains a bacterial origin of replication ("ori"), an IPTG inducible promoter, a ribosome binding site ("RBS"), a 6-His tag and restriction enzyme sites. The amplified IL-19 protein DNA and the vector pQE60 both were digested with *Nco*I and *Bgl*II and the digested DNAs were then ligated together. Insertion of the IL-19 protein DNA into the restricted pQE60 vector placed the IL-19 protein coding region downstream of and operably linked to the vector's IPTG-inducible promoter and in-frame with an initiating ATG appropriately positioned for translation of IL-19 protein.

The ligation mixture was transformed into competent *E. coli* cells using standard procedures. Such procedures are described in Sambrook et al., MOLECULAR CLONING: A LABORATORY MANUAL, 2nd Ed.; Cold Spring Harbor Laboratory Press, Cold Spring Harbor, N.Y. (1989). *E. coli* strain M 15/rep4, containing multiple copies of the plasmid pREP4, which expresses lac repressor and confers kanamycin resistance ("Kan"'), was used in carrying out the illustrative example described here. This strain, which is only one of many that are suitable for expressing IL-19 protein, is available commercially from Qiagen.

Transformants were identified by their ability to grow on LB plates in the presence of ampicillin and kanamycin. Plasmid DNA was isolated from resistant colonies and the identity of the cloned DNA was confirmed by restriction analysis.

Clones containing the desired constructs were grown overnight ("O/N") in liquid culture in LB media supplemented with both ampicillin (100 µg/ml) and kanamycin (25 µg/ml).

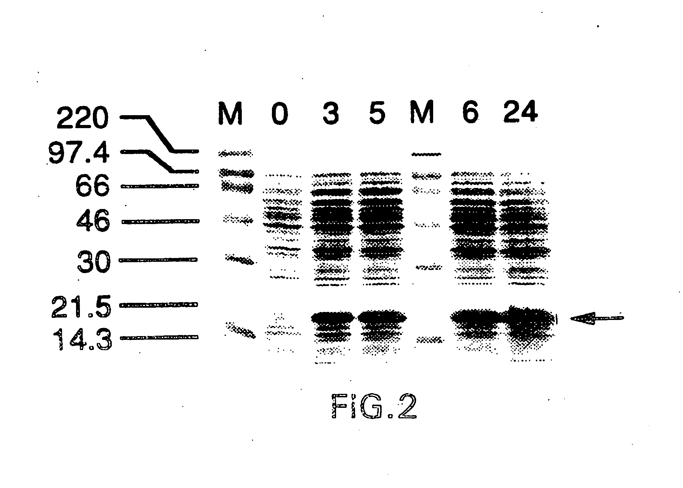

The O/N culture was used to inoculate a large culture, at a dilution of approximately 1:100 to 1:250. The cells were grown to an optical density at 600nm ("OD600") of between 0.4 and 0.6. Isopropyl-B-D-thiogalactopyranoside ("IPTG") was then added to a final concentration of 0.1 mM to induce transcription from lac repressor sensitive promoters, by inactivating the *lac*I repressor. Cells subsequently were incubated further for 4 hours (Figure 2 shows IL-19 protein induction; samples removed 0, 3, 5, 6, and 24 hours after the addition of IPTG were run on a 12.5% polyacrylamide gel and stained with brilliant blue). The mobility of the IL-19 protein is indicated by an arrow. Cells were then harvested by centrifugation and disrupted by gentle shaking overnight in 6M guanidine HCl in 50 mM NaPO₄ buffer at pH 8.0 at 4°C. The lysate was then centrifuged and passed over a Sepharose CL-4B (Pharmacia) column. The flowthrough was then passed over a column containing activated Ni²⁺-NTA-agarose (Qiagen). IL-19 protein was collected from the column in a fraction consisting of 6M guanidine HCl pH 5.0. Guanidine HCl was removed from the IL-19-containing fraction by dialysis against successively reduced concentrations of guanidine in phosphate-buffered saline (PBS) at pH 5.5.

### Example 2: Cloning and Expression of IL-19 in a Baculovirus Expression System

The cDNA sequence encoding the full length IL-19 protein in the deposited clone was amplified using PCR oligonucleotide primers corresponding to the 5' and 3' sequences of the gene:

The 5' primer had the sequence 5' GGC GGG ATC CCG CCA TCA TGA AGT TAC AGT GTG TTT CCC 3' [SEQ ID NO:6] containing the underlined *Bam*HI restriction enzyme site followed by 29 bases of the sequence of IL-19 protein in Figure 1. Inserted into an expression vector, as described below, the 5' end of the amplified fragment encoding IL-19 provided an efficient signal peptide. An efficient signal for initiation of translation in eukaryotic cells, as described by Kozak, M., J. Mol. Biol. 196: 947-950 (1987) is appropriately located in the vector portion of the construct.

The 3' primer had the sequence 5'CCC AAG CTT GGT ACC TCA TCA AGC TGA GGA CAT TAC 3' [SEQ ID NO:7] containing the underlined *Asp*718 restriction site followed by nucleotides complementary to the last 21 nucleotides of the IL-19 coding sequence set out in Figure 1.

The amplified fragment was isolated from a 1% agarose gel using a commercially available kit ("Geneclean," BIO 101 Inc., La Jolla, Ca.). The fragment then was digested with *Bam*HI and *Asp*718 and again was purified on a 1% agarose gel. This fragment is designated herein F2.

The vector and pA2-GP were used to express the IL-19 protein in the baculovirus expression system, using standard methods, as described in Summers et al, A MANUAL OF METHODS FOR BACULOVIRUS VECTORS AND INSECT CELL CULTURE PROCEDURES, Texas Agricultural Experimental Station Bulletin No. 1555 (1987). This expression vector contains the strong polyhedrin promoter of the *Autographa californica* nuclear polyhedrosis virus (AcMNPV) followed by convenient restriction sites. The signal peptide of AcMNPV gp67, including the N-terminal methionine, is located just upstream of a BamHI site. The polyadenylation site of the simian virus 40 ("SV40") is used for efficient polyadenylation. For an easy selection of recombinant virus the beta-galactosidase gene from *E. coli* is inserted in the same orientation as the polyhedrin promoter and is followed by the polyadenylation signal of the polyhedrin gene. The polyhedrin sequences are flanked at both sides by viral sequences for cell-mediated homologous recombination with wild-type viral DNA to generate viable virus that express the cloned polynucleotide. The IL-19 protein was also expressed in baculovirus using the pA2 vector.

Many other baculovirus vectors could be used in place of pA2-GP, such as pAc373, pVL941 and pAcIM1 provided, as those of skill readily will appreciate, that construction provides appropriately located signals for transcription, translation, trafficking and the like, such as an in-frame AUG and a signal peptide, as required. Such vectors are described in Luckow et al., Virology 170: 31-39, among others.

The plasmid was digested with the restriction enzymes *Bam*HI and *Asp*718 and then was dephosphorylated using calf intestinal phosphatase, using routine procedures known in the art. The DNA was then isolated from a 1% agarose gel using a commercially available kit ("Geneclean" BIO 101 Inc., La Jolla, Ca.). This vector DNA is designated herein "V2".

Fragment F2 and the dephosphorylated plasmid V2 were ligated together with T4 DNA ligase. *E. coli* HB101 cells were transformed with ligation mix and spread on culture plates. Bacteria were identified that contained the plasmid with the human IL-19 gene by digesting DNA from individual colonies using *Bam*HI and *Asp*718 and then analyzing the digestion product by gel electrophoresis. The sequence of the cloned fragment was confirmed by DNA sequencing. This plasmid is designated herein pBacIL-19.

5 µg of the plasmid pBacIL-19 was co-transfected with 1.0 µg of a commercially available linearized baculovirus DNA ("BaculoGold^{™} baculovirus DNA", Pharmingen, San Diego, CA.), using the lipofection method described by Felgner et al., Proc. Natl. Acad. Sci. USA 84: 7413-7417 (1987). 1µg of BaculoGold™ virus DNA and 5 µg of the plasmid pBacCKβ-15 were mixed in a sterile well of a microtiter plate containing 50 µl of serum-free Grace's medium (Life. Technologies Inc., Gaithersburg, MD). Afterwards 10 µl Lipofectin plus 90 µl Grace's medium are added, mixed and incubated for 15 minutes at room temperature. Then the transfection mixture was added drop-wise to Sf9 insect cells (ATCC CRL 1711) seeded in a 35 mm tissue culture plate with 1 ml Grace's medium without serum. The plate was rocked back and forth to mix the newly added solution. The plate was then incubated for 5 hours at 27°C. After 5 hours the transfection solution was removed from the plate and 1 ml of Grace's insect medium supplemented with 10% fetal calf serum was added. The plate was put back into an incubator and cultivation is continued at 27°C for four days.

After four days the supernatant was collected and a plaque assay is performed, as described by Summers and Smith, cited above. An agarose gel with "Blue Gal" (Life Technologies Inc., Gaithersburg) was used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10).

Four days after serial dilution, the virus was added to the cells. After appropriate incubation, blue stained plaques were picked with the tip of an Eppendorf pipette. The agar containing the recombinant viruses was then resuspended in an Eppendorf tube containing 200 µl of Grace's medium. The agar was removed by a brief centrifugation and the supernatant containing the recombinant baculovirus was used to infect Sf9 cells seeded in 35 mm dishes. Four days later the supernatants of these culture dishes were harvested and then they were stored at 4°C. A clone containing properly inserted hESSB I, II and III were identified by DNA analysis including restriction mapping and sequencing. This is designated herein as V-IL-19.

Sf9 cells were grown in Grace's medium supplemented with 10% heat-inactivated FBS. The cells were infected with the recombinant baculovirus V-IL-19 at a multiplicity of infection ("MOI") of about 2 (about I to about 3). Six hours later the medium was removed and was replaced with SF900 II medium minus methionine and cysteine (available from Life Technologies Inc., Gaithersburg). 42 hours later, 5 µCi of ³⁵S-methionine and 5 µCi ³⁵S-cysteine (available from Amersham) were added. The cells were further incubated for 16 hours and then they were harvested by centrifugation, lysed and the labeled proteins were visualized by SDS-PAGE and autoradiography.

### Example 3: In vitro transcription/translation of IL-19 protein

Recombinant IL-19 proteins were prepared using the TNT Coupled Wheat Germ Extract System (Promega, Madison, WI).

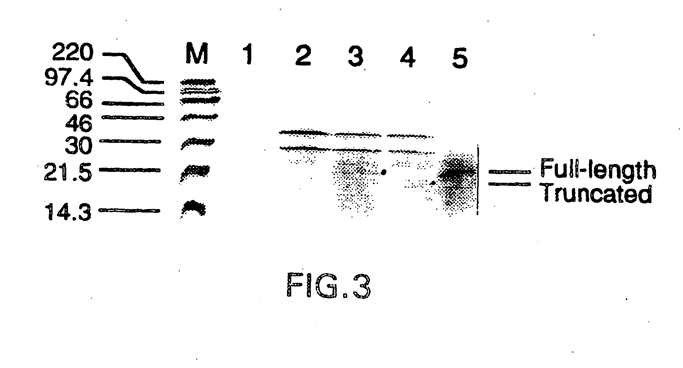

25 µl of TNT wheat germ extract, 10 U T3 RNA polymerase, 1 mM amino acid mixture (methionine-free), 4 µCi ³⁵S-methionine (1000 Ci/mmol), 40 U RNasin (Promega, Madison, WI), and 1 µg template DNA were combined in a final volume of 50 µl and incubated at 30°C for 2h. Coupled transcription/translation reactions included the following template DNAs: (1) No DNA, (2) pBluescript, (3) nucleotides 44-577 (corresponding to amino acids 1-177) of the IL-19 sequence shown in Figure 1 cloned into pBluescript, (4) nucleotides 116-577 (corresponding to amino acids 25-177 shown in Figure 1) of the IL-19 coding sequence cloned into pBluescript, (5) a gel-purified PCR product derived from the template described in (3) and amplified using M13 forward and reverse primers in a standard PCR reaction. Samples were heated to 95°C for 10 minutes and a 5 µl aliquot of each sample was then loaded on to a 15% polyacrylamide gel. The gel was run at 100 volts for approximately 2 hours. The gel was then dried and exposed to X-ray film for 3 days at room temperature. The mobility of full-length and truncated (no signal sequence) IL-19 proteins are indicated in Figure 3. An apparent molecular mass marker (M) shows the relative mobilities of 14.3, 21.5, 30, 46, 66, 97.4, and 220 kD proteins.

### Example 4: Cloning and Expression in Mammalian Cells

Most of the vectors used for the transient expression of the IL-19 protein gene sequence in mammalian cells should carry the SV40 origin of replication. This allows the replication of the vector to high copy numbers in cells (e.g. COS cells) which express the T antigen required for the initiation of viral DNA synthesis. Any other mammalian cell line can also be utilized for this purpose.

A typical mammalian expression vector contains the promoter element, which mediates the initiation of transcription of mRNA, the protein coding sequence, and signals required for the termination of trancription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRs) from Retroviruses, e.g. RSV, HTLVI, HIVI and the early promoter of the cytomegalovirus (CMV). However, cellular signals can also be used (e.g. human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Mammalian host cells that could be used include, human Hela, 283, H9 and Jurkart cells, mouse NIH3T3 and C127 cells, Cos 1, Cos 7 and CV1, African green monkey cells, quail QC1-3 cells, mouse L cells and Chinese hamster ovary cells.

Alternatively, the gene can be expressed in stable cell lines that contain the gene integrated into a chromosome. The co-transfection with a selectable marker such as dhfr, gpt, neomycin, hygromycin allows the identification and isolation of the transfected cells

The transfected gene can also be amplified to express large amounts of the encoded protein. The DHFR (dihydrofolate reductase) is a useful marker to develop cell lines that carry several hundred or even several thousand copies of the gene of interest. Another useful selection marker is the enzyme glutamine synthase (GS) (Murphy et al., Biochem J. 227:277-279 (1991); Bebbington et al., Bio/Technology 10:169-175 (1992)). Using these markers, the mammalian cells are grown in selective medium and the cells with the highest resistance are selected. These cell lines contain the amplified gene(s) integrated into a chromosome. Chinese hamster ovary (CHO) cells are often used for the production of proteins.

The expression vectors pC1 and pC4 contain the strong promoter (LTR) of the Rous Sarcoma Virus (Cullen et al., Molecular and Cellular Biology, 438-4470 (March, 1985)) plus a fragment of the CMV-enhancer (Boshart et al., Cell 41:521-530 (1985)). Multiple cloning sites, e.g. with the restriction enzyme cleavage sites BamHI, XbaI and Asp718, facilitate the cloning of the gene of interest. The vectors contain in addition the 3' intron, the polyadenylation and termination signal of the rat preproinsulin gene.

### Example 4(a): Cloning and Expression in COS Cells

The expression plasmid, pIL-19 HA, is made by cloning a cDNA encoding IL-19 into the expression vector pcDNA4 (which can be obtained from Invitrogen, Inc.).

The expression vector pcDNA4 contains: (1) an *E.coli* origin of replication effective for propagation in *E. coli* and other prokaryotic cells; (2) an ampicillin resistance gene for selection of plasmid-containing prokaryotic cells; (3) an SV40 origin of replication for propagation in eukaryotic cells; (4) a CMV promoter, a polylinker, an SV40 intron, and a polyadenylation signal arranged so that a cDNA conveniently can be placed under expression control of the CMV promoter and operably linked to the SV40 intron and the polyadenylation signal by means of restriction sites in the polylinker.

A DNA fragment encoding the IL-19 protein and an HA tag fused in frame to its 3' end is cloned into the polylinker region of the vector so that recombinant protein expression is directed by the CMV promoter. The HA tag corresponds to an epitope derived from the influenza hemagglutinin protein described by Wilson et al., Cell 37: 767 (1984). The fusion of the HA tag to the target protein allows easy detection of the recombinant protein with an antibody that recognizes the HA epitope.

The plasmid construction strategy is as follows. The IL-19 cDNA of the deposited clone is amplified using primers that contain convenient restriction sites, much as described above regarding the construction of expression vectors for expression of IL-19 in *E. coli.* To facilitate detection, purification and characterization of the expressed IL-19, one of the primers contains a hemagglutinin tag ("HA tag") as described above.

Suitable primers include the following, which are used in this example. The 5' primer, containing the underlined BamHI site, an AUG start codon and 22 bp of the 5' coding region has the following sequence:
5'GGC GGG ATC_CCG CCA TGA AGT TAG AGT GTG TTT CCC 3' (SEQ ID NO:8).

The 3' primer, containing the underlined Asp 718 site, a stop codon, 10 codons whereafter forming the hemagglutinin HA tag, and 25 bp of 3' coding sequence (at the 3' end) has the following sequence:
5' CCC AAG CTT GGT ACC TCA TCA GAA AGC GTA GTC TGG GAC GTC GTA TGG GTA AGC TGA GGA CAT TAC TTC ATG ATT C 3' (SEQ ID NO:9).

The PCR amplified DNA fragment and the vector, pcDNAI/Amp, are digested with HindIII and XhoI and then ligated. The ligation mixture is transformed into *E. coli* strain SURE (available from Stratagene Cloning Systems, 11099 North Torrey Pines Road, La Jolla, CA 92037), and the transformed culture is plated on ampicillin media plates which then are incubated to allow growth of ampicillin resistant colonies. Plasmid DNA is isolated from resistant colonies and examined by restriction analysis and gel sizing for the presence of the IL-19-encoding fragment.

For expression of recombinant IL-19, COS cells are transfected with an expression vector, as described above, using DEAE-DEXTRAN, as described, for instance, in Sambrook et al., MOLECULAR CLONING: A LABORATORY MANUAL, Cold Spring Laboratory Press, Cold Spring Harbor, New York. (1989). Cells are incubated under conditions for expression of IL-19 by the vector.

Expression of the IL-19/HA fusion protein is detected by radiolabelling and immunoprecipitation, using methods described in, for example Harlow et al., ANTIBODIES: A LABORATORY MANUAL, 2nd Ed.; Cold Spring Harbor Laboratory Press, Cold Spring Harbor, New York (1988). To this end, two days after transfection, the cells are labeled by incubation in media containing ³⁵S-cysteine for 8 hours. The cells and the media are collected, and the cells are washed and the lysed with detergent-containing RIPA buffer: 150 mM NaCl, 1% NP-40, 0.1% SDS, 1% NP-40, 0.5% DOC, 50 mM TRIS, pH 7.5, as described by Wilson et al. cited above. Proteins are precipitated from the cell lysate and from the culture media using an HA-specific monoclonal antibody. The precipitated proteins then are analyzed by SDS-PAGE gels and autoradiography. An expression product of the expected size is seen in the cell lysate, which is not seen in negative controls.

### Example 4(b): Cloning and Expression in CHO Cells

The vector pC4 is used for the expression of IL-19 protein. Plasmid pC4 is a derivative of the plasmid pSV2-dhfr [ATCC Accession No. 37146]. Both plasmids contain the mouse DHFR gene under control of the SV40 early promoter. Chinese hamster ovary- or other cells lacking dihydrofolate activity that are transfected with these plasmids can be selected by growing the cells in a selective medium (alpha minus MEM, Life Technologies) supplemented with the chemotherapeutic agent methotrexate. The amplification of the DHFR genes in cells resistant to methotrexate (MTX) has been well documented (see, e.g., Alt, F.W., Kellems, R.M., Bertino, J.R., and Schimke, R.T., 1978, J. Biol. Chem. 253:1357-1370, Hamlin, J.L. and Ma, C. 1990, Biochem. et Biophys. Acta, 1097:107-143, Page, M.J. and Sydenham, M.A. 1991, Biotechnology Vol. 9:64-68). Cells grown in increasing concentrations of MTX develop resistance to the drug by overproducing the target enzyme, DHFR, as a result of amplification of the DHFR gene. If a second gene is linked to the DHFR gene it is usually co-amplified and over-expressed. It is state of the art to develop cell lines carrying more than 1,000 copies of the genes. Subsequently, when the methotrexate is withdrawn, cell lines contain the amplified gene integrated into the chromosome(s).

Plasmid pC4 contains for the expression of the gene of interest a strong promoter of the long terminal repeat (LTR) of the Rouse Sarcoma Virus (Cullen, et al., Molecular and Cellular Biology, March 1985:438-4470) plus a fragment isolated from the enhancer of the immediate early gene of human cytomegalovirus (CMV) (Boshart et al., Cell 41:521-530, 1985). Downstream of the promoter are the following single restriction enzyme cleavage sites that allow the integration of the genes: BamHI, PvuII, and NruI. Behind these cloning sites the plasmid contains translational stop codons in all three reading frames followed by the 3' intron and the polyadenylation site of the rat preproinsulin gene. Other high efficient promoters can also be used for the expression, e.g., the human β-actin promoter, the SV40 early or late promoters or the long terminal repeats from other retroviruses, e.g., HIV and HTLVI. For the polyadenylation of the mRNA other signals, e.g., from the human growth hormone or globin genes can be used as well.

Stable cell lines carrying a gene of interest integrated into the chromosomes can also be selected upon co-transfection with a selectable marker such as gpt, G418 or hygromycin. It is advantageous to use more than one selectable marker in the beginning, e.g., G418 plus methotrexate.

The plasmid pC4 is digested with the restriction enzyme BamHI and then dephosphorylated using calf intestinal phosphates by procedures known in the art. The vector is then isolated from a 1% agarose gel.

The DNA sequence encoding IL-19 protein is amplified using PCR oligonucleotide primers specific to the amino terminal sequence of the IL-19 protein and to carboxy terminal sequence 3' to the gene. Additional nucleotides containing restriction sites to facilitate cloning are added to the 5' and 3' sequences respectively.

The 5' primer has the sequence 5' GGC GGG ATC CCG CCA TCA TGA AGT TAC AGT GTG TTT CCC 3' (SEQ ID NO: 10), containing the underlined BamHI restriction enzyme site followed by Kozak sequence and 23 bases of the sequence of IL-19 in FIG. 1. The 3' primer has the sequence 5' CCC AAG CTT GGT ACC TCA TGA AGC TGA GGA CAT TAC 3' (SEQ ID NO: 11), containing the underlined Asp 718 restriction site followed by nucleotides complementary to 15 bp of the nucleotide sequence preceding the stop codon in FIG. 1. The restrictions sites are convenient to restriction enzyme sites in the CHO expression vector pC4.

The amplified fragments are isolated from a 1% agarose gel as described above and then digested with the endonucleases BamHI and Asp718 and then purified again on a 1% agarose gel.

The isolated fragment and the dephosphorylated vector are then ligated with T4 DNA ligase. *E. coli* HB101 cells are then transformed and bacteria identified that contained the plasmid pC4 inserted in the correct orientation using the restriction enzyme BamHI. The sequence of the inserted gene is confirmed by DNA sequencing.

### Transfection of CHO-DHFR-cells

Chinese hamster ovary cells lacking an active DHFR enzyme are used for transfection. 5 µg of the expression plasmid pC4 are cotransfected with 0.5 µg of the plasmid pSVneo using the lipofecting method (Felgner *et al., supra*). The plasmid pSV2-neo contains a dominant selectable marker, the gene neo from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) and cultivated from 10-14 days. After this period, single clones are trypsinized and then seeded in 6-well petri dishes using different concentrations of methotrexate (25 nM, 50 nM, 100 nM, 200 nM, 400 nM). Clones growing at the highest concentrations of methotrexate are then transferred to new 6-well plates containing even higher concentrations of methotrexate (500 nM, 1 µM, 2 µM, 5 µM). The same procedure is repeated until clones grow at a concentration of 100 µM.

The expression of the desired gene product is analyzed by Western blot analysis and SDS-PAGE.

### Example 5: Tissue distribution of IL-1 9 protein expression

Northern blot analysis was carried out to examine the levels of expression of IL-19 protein in human tissues, using methods described by, among others, Sambrook et al, cited above.

HL-60, THP-1 and U937 cell lines and primary human monocytes isolated by adherence from a mixed leukocyte population were grown for 12 hours either in the presence (+) or absence (-) of bacterial lipopolysaccharide (LPS). Total RNA was prepared from the cultures with TRIzol Reagent (Life Technologies, Gaithersburg, MD) essentially as described by the manufacturer. Total RNA (10 µg) was dried completely, resuspended in a formamide/formaldehyde loading buffer, and resolved by electrophoresis through a 1% agarose gel containing 2.2 M formaldehyde. The gel was transferred overnight in 20X SSC to a nylon membrane (Boehringer Mannheim, Indianapolis, IN) Probe DNA was prepared by PCR amplifying the entire IL-19 insert shown in Figure 1 using M13 Forward and Reverse primers. Probe DNA (25 ng) was labeled with 32P using the RediPrime Random Primer Labeling Kit (Amersham Life Science) to a specific activity of greater than 10⁵ CPM/ng. The blot was hybridized with denatured probe in al 0 ml Hybrizol hybridization solution overnight at 42°C. The blot was then washed in approximately 100 ml of 0.2X SSC/0.1% SDS at 25 °C for 20 minutes, and then twice in approximately 100 ml of 0.2X SSC/0.1% SDS at 65 °C for 15 min. The biot was then exposed to x-ray film for 5 days, and is shown in Figure 4. The arrow shows the migration of the IL-19-specific RNA. An RNA marker shows the relative mobilities of 0.24, 1.35, 2.37, 4.40, 7.45, and 9.49 kb RNAs.

It will be clear that the invention may be Practiced otherwise than as particularly described in the foregoing description and examples.

Numerous modifications and variations of the present invention are possible in light of the above teachings and, therefore, are within the scope of the appended claims.

## Claims

1. A polynucleotide selected from the group consisting of:
(a) a polynucleotide encoding residues 1 to 153 of SEQ ID NO:2;
(b) a polynucleotide encoding residues -24 to 153 of SEQ ID NO:2;
(c) a polynucleotide having the sequence of nucleotides 116 to 574 of SEQ ID NO:1;
(d) a polynucleotide having the sequence of nucleotides 44 to 574 of SEQ ID NO:1;
(e) a polynucleotide encoding the mature form of the polypeptide encoded by the cDNA contained in ATCC 97662;
(f) a polynucleotide encoding the full length polypeptide encoded by the cDNA contained in ATCC 97662;
(g) a polynucleotide having the coding sequence of the cDNA contained in ATCC 97662 encoding the mature form of the polypeptide;
(h) a polynucleotide having the coding sequence of the cDNA contained in ATCC 97662 encoding the full length polypeptide;
(i) a polynucleotide encoding an immunogenic epitope-bearing portion of a polypeptide encoded by a polynucleotide of any one of (a) to (h), wherein said immunogenic epitope is a portion of the polypeptide that elicits an antibody response when the whole polypeptide is the immunogen;
(j) a polynucleotide encoding an epitope-bearing portion of a IL-19 polypeptide comprising amino acid residues VDNHGLRRC or RVFKDHQEPNPKILRKISS;
(k) a polynucleotide at least 90% identical to the polynucleotide as defined in any one of (a) to (h) and which encodes a polypeptide having IL-19 activity, wherein said activity is the property of modulating the synthesis of at least one cytokine in the group consisting of IFN-γ, lymphotoxin, IL-2, IL-3 and GM-CSF in a population of T helper cells induced to synthesize one or more of these cytokines by exposure to syngeneic antigen presenting cells (APCs) and antigen; and
(l) a polynucleotide at least 90% identical to the polynucleotide as defined in any one of (a) to (h), wherein the complementary strand of said polynucleotide hybridizes under stringent hybridization conditions with the polynucleotide of any one of (a) to (h);
or the complementary strand of such a polynucleotide.

2. The polynucleotide of claim 1, which is DNA or RNA.

3. The polynucleotide of claim 2, which is genomic DNA.

4. The polynucleotide of any one of claims 1 to 3 fused to a heterologous polynucleotide.

5. The polynucleotide of claim 4, wherein the heterologous polynucleotide encodes a heterologous polypeptide.

6. The polynucleotide, of claim 5 wherein the heterologous polypeptide is fused to a polypeptide encoded by the polynucleotide of any one of claims 1 to 3.

7. A method of making a recombinant vector comprising inserting a polynucleotide of any one of claims 1 to 6 into a vector.

8. A vector containing the polynucleotide of any one of claims 1 to 6 or produced by the method of claim 7.

9. The vector of claim 8 in which the polynucleotide is operatively linked to expression control sequences allowing expression in prokaryotic or eukaryotic host cells.

10. A method of making a recombinant host cell comprising introducing the vector of claim 8 or 9 into a host cell.

11. A host cell genetically engineered with the polynucleotide of any one of claims 1 to 6 or the vector of claim 8 or 9 or produced by the method of claim 10.

12. The host cell of claim 11, wherein said cell is a prokaryotic cell, eukaryotic cell, animal cell, mammalian cell, insect cell, plant cell, fungal cell, COS cell, CHO cell, or E. coli cell.

13. A process for producing a polypeptide having IL-19 activity, wherein said activity is the property of modulating the synthesis of at least one cytokine in the group consisting of IFN-γ, lymphotoxin, IL-2, IL-3, and GM-CSF in a population of T helper cells induced to synthesize one or more of these cytokines by exposure to syngeneic antigen-presenting cells (APCs) and antigen, comprising: culturing the host cell of claim 11 or 12 under conditions such that said polypeptide is expressed and recovering the polypeptide encoded by said polynucleotide from the culture.

14. A polypeptide having the amino acid sequence encoded by a polynucleotide of any one of claims 1 to 6 or obtainable by the process of claim 13.

15. A polypeptide selected from the group consisting of:
(a) a polypeptide having an amino acid sequence at least 80% identical to amino acid residues -24 to 153 of SEQ ID NO:2, wherein said polypeptide has IL-19 activity;
(b) a polypeptide having an amino acid sequence at least 80% identical to amino acid residues 1 to 153 of SEQ ID NO:2, wherein said polypeptide has IL-19 activity;
(c) a polypeptide having an amino acid sequence at least 80% identical to the full length polypeptide encoded by the cDNA contained in ATCC 97662, wherein said polypeptide has IL-19 activity;
(d) a polypeptide having an amino acid sequence at least 80% identical to the mature form of the polypeptide encoded by the cDNA contained in ATCC 97662, wherein said polypeptide has IL-19 activity;
(e) a polypeptide consisting of at least 30 contiguous amino acids of amino acid residues -24 to 153 of SEQ ID NO:2, wherein said polypeptide has IL-19 activity;
(f) a polypeptide consisting of at least 50 amino acids of amino acid residues -24 to 153 of SEQ ID NO:2, wherein said polypeptide has IL-19 activity;
(g) a polypeptide consisting of at least 30 amino acids of the full length polypeptide encoded by the cDNA contained in ATCC 97662, wherein said polypeptide has IL-19 activity; and
(h) a polypeptide consisting of at least 50 amino acids of the full length polypeptide encoded by the cDNA contained in ATCC 97662, wherein said polypeptide has IL-19 activity, wherein said IL-19 activity of any one of (a) to (h) is the property of modulating the synthesis of at least one cytokine in the group consisting of IFN-γ, lymphotoxin, IL-2, IL-3, and GM-CSF in a population of T helper cells induced to synthesize one or more of these cytokines by exposure to syngeneic antigen-presenting cells (APCs) and antigen.

16. The polypeptide of either claim 14 or 15, wherein said polypeptide is radiolabeled or glycosylated.

17. The polypeptide of any one of claims 13 to 15, wherein said polypeptide is fused to a heterologous polypeptide.

18. The polypeptide of any one of claims 14 to 17, wherein said polypeptide lacks an N-terminal methionine.

19. An antibody that specifically binds to the polypeptide of any one of claims 14 to 16.

20. The antibody of claim 19, wherein said antibody is polyclonal or monoclonal.

21. A nucleic acid molecule comprising a polynucleotide, which hybridizes under stringent hybridization conditions to a polynucleotide of any one of claims 1 to 3, wherein the complementary strand of said polynucleotide which hybridizes is at least 90% identical to a polynucleotide of any one of claims 1 to 3.

22. An antagonist of the polypeptide of either claim 14 or 15 wherein said antagonist is the antibody of either claim 19 or 20.

23. A pharmaceutical composition comprising the polypeptide of any one of claims 14 to 18, the antibody of either claim 19 or 20, or the antagonist of claim 22, and optionally a pharmaceutically acceptable carrier.

24. A diagnostic composition comprising the polynucleotide of any one of claims 1 to 3 or the antibody of either claim 19 or 20.

25. Use of the polypeptide of any one of claims 14 to 18 for the preparation of a pharmaceutical composition for treatment of an individual in need of decreased levels of IFN-γ, TNF-α and/or IL-6, of rheumatoid arthritis, systemic lupus erythematosus (SLE), myasthenia gravis, insulin-dependent diabetes mellitus, thyroiditis, parasitic infections or for "Adoptive Immunotherapy" of cancer.

26. Use of the antagonist of claim 22 or the antibody of claim 19 or 20 for the preparation of a pharmaceutical composition for increasing IL-2 production.

27. Use of the polypeptide of any one of claims 14 to 18. in the preparation of a medicament.

28. Use of the antibody of any one of claims 19 or 20 in the preparation of a medicament.

29. Use of the antagonist of claim 22 in the preparation of a medicament.

30. A pharmaceutical composition for the treatment of an individual in need of decreased levels of IFN-γ, TNF-α and/or IL-6, of rheumatoid arthritis, systemic lupus erythematosus (SLE), myasthenia gravis, insulin-dependent diabetes mellitus, thyroiditis, parasitic infections or for "Adoptive Immunotherapy" of cancer, comprising the polypeptide of anyone of claims 14 to 18.

31. A pharmaceutical composition for increasing IL-2 production, comprising the antagonist of claim 22 or the antibody of claim 19 or 20.

## Patentansprüche

1. Polynucleotid, ausgewählt aus der Gruppe bestehend aus:
(a) einem Polynucleotid, welches die Reste 1 bis 153 der SEQ ID NO:2 codiert;
(b) einem Polynucleotid, welches die Reste -24 bis 153 der SEQ ID NO:2 codiert;
(c) einem Polynucleotid, welches die Nucleotidsequenz 116 bis 574 der SEQ ID NO:1 aufweist;
(d) einem Polynucleotid, welches die Nucleotidsequenz 44 bis 574 der SEQ ID NO:1 aufweist;
(e) einem Polynucleotid, welches die reife Form des Polypeptids codiert, welches von der cDNA codiert wird, die in ATCC 97662 enthalten ist;
(f) einem Polynucleotid, welches das Polypeptid in voller Länge codiert, welches von der cDNA codiert wird, die in ATCC 97662 enthalten ist;
(g) einem Polynucleotid, welches die codierende Sequenz der cDNA aufweist, welche in ATCC 97662 enthalten ist, das die reife Form des Polypeptids codiert;
(h) einem Polynucleotid, welches die codierende Sequenz der cDNA aufweist, welche in ATCC 97662 enthalten ist, das das Polypeptid in voller Länge codiert;
(i) einem Polynucleotid, welches einen immunogenen Epitop-tragenden Teil von einem Polypeptid codiert, welches von einem Polynucleotid nach einem von (a) bis (h) codiert wird, wobei das immunogene Epitop ein Teil des Polypeptids ist, welches eine Antikörperantwort hervorruft, wenn das gesamte Polypeptid das Immunogen ist;
(j) einem Polynucleotid, welches ein Epitop-tragenden Teil von einem IL-19-Polypeptid codiert, welches die Aminosäurereste VDNHGLRRC oder RVFKDHQEPNPKILRKISS umfasst;
(k) einem Polynucleotid, welches zu mindestens 90% identisch mit dem Polynucleotid ist, wie definiert in einem von (a) bis (h), und welches ein Polypeptid mit IL-19-Aktivität codiert, wobei die Aktivität die Eigenschaft einer Modulation der Synthese von mindestens einem Cytokin in der Gruppe bestehend aus IFN-γ, Lymphotoxin, IL-2, IL-3 und GM-GSF in einer Population von T-Helferzellen ist, die induziert ist, um eines oder mehrere von diesen Cytokinen zu synthetisieren, indem sie syngenen Antigen-präsentierenden Zellen (APCs) und Antigen ausgesetzt wird; und
(l) einem Polynucleotid, welches zu mindestens 90% identisch mit dem Polynucleotid ist, wie definiert in einem von (a) bis (h), wobei der komplementäre Strang des Polynucleotids unter stringenten Hybrisierungsbedingungen mit dem Polynucleotid nach einem von (a) bis (h) hybridisiert;
oder der komplementäre Strang eines solchen Polynucleotids.

2. Polynucleotid nach Anspruch 1, welches DNA oder RNA ist.

3. Polynucleotid nach Anspruch 2, welches genomische DNA ist.

4. Polynucleotid nach einem der Ansprüche 1 bis 3, welches an ein heterologes Polynucleotid fusioniert ist.

5. Polynucleotid nach Anspruch 4, wobei das heterologe Polynucleotid ein heterologes Polypeptid codiert.

6. Polynucleotid nach Anspruch 5, wobei das heterologe Polypeptid an ein Polypeptid fusioniert ist, welches von dem Polynucleotid nach einem der Ansprüche 1 bis 3 codiert wird.

7. Verfahren zur Herstellung eines rekombinanten Vektors, welches das Inserieren eines Polynucleotids nach einem der Ansprüche 1 bis 6 in einen Vektor umfasst.

8. Vektor, welcher das Polynucleotid nach einem der Ansprüche 1 bis 6 enthält oder durch das Verfahren nach Anspruch 7 hergestellt wird.

9. Vektor nach Anspruch 8, in welchem das Polynucleotid funktionell verknüpft ist mit Expressionskontrollsequenzen, die die Expression in prokaryontischen oder eukaryontischen Wirtszellen erlauben.

10. Verfahren zur Herstellung einer rekombinanten Wirtszelle, welches die Einführung des Vektors nach Anspruch 8 oder 9 in eine Wirtszelle umfasst.

11. Wirtszelle, gentechnisch verändert mit dem Polynucleotid nach einem der Ansprüche 1 bis 6 oder dem Vektor nach Anspruch 8 oder 9 oder hergestellt durch das Verfahren nach Anspruch 10.

12. Wirtszelle nach Anspruch 11, wobei die Zelle eine prokaryontische Zelle, eukaryontische Zelle, tierische Zelle, Säugerzelle, Insektenzelle, Pflanzenzelle, Pilzzelle, COS-Zelle, CHO-Zelle oder E.coli-Zelle ist.

13. Verfahren zur Herstellung eines Polypeptids mit IL-19-Aktivität, wobei die Aktivität die Eigenschaft einer Modulation der Synthese von mindestens einem Cytokin in der Gruppe bestehend aus IFN-γ, Lymphotoxin, IL-2, IL-3 und GM-GSF in einer Population von T-Helferzellen ist, die induziert ist, um eines oder mehrere von diesen Cytokinen zu synthetisieren, indem sie syngenen Antigen-präsentierenden Zellen (APCs) und Antigen ausgesetzt wird, umfassend: Züchten der Wirtszelle nach Anspruch 11 oder 12 unter Bedingungen, so dass das Polypeptid exprimiert wird, und Gewinnen des Polypeptids, welches von dem Polynucleotid aus der Kultur codiert wird.

14. Polypeptid, welches die Aminosäuresequenz aufweist, die von dem Polynucleotid nach einem der Ansprüche 1 bis 6 codiert wird oder durch das Verfahren nach Anspruch 13 erhältlich ist.

15. Polypeptid ausgewählt aus der Gruppe bestehend aus:
(a) einem Polypeptid, welches eine Aminosäuresequenz aufweist, welche zu mindestens 80% identisch mit den Aminosäureresten -24 bis 153 von SEQ ID NO:2 ist, wobei das Polypeptid IL-19-Aktivität aufweist;
(b) einem Polypeptid, welches die Aminosäuresequenz aufweist, welche zu mindestens 80% identisch mit den Aminosäureresten 1 bis 153 von SEQ ID NO:2 ist, wobei das Polypeptid IL-19-Aktivität aufweist;
(c) einem Polypeptid, welches die Aminosäuresequenz aufweist, welche zu mindestens 80% identisch mit dem Polypeptid in voller Länge ist, welches von der cDNA enthalten in ATCC 97662 codiert wird, wobei das Polypeptid IL-19-Aktivität aufweist;
(d) einem Polypeptid, welches die Aminosäuresequenz aufweist, welche zu mindestens 80% identisch mit der reifen Form des Polypeptids ist, welches von der cDNA enthalten in ATCC 97662 codiert wird, wobei das Polypeptid IL-19-Aktivität aufweist;
(e) einem Polypeptid bestehend aus mindestens 30 zusammenhängenden Aminosäuren der Aminosäurereste -24 bis 153 von SEQ ID NO:2, wobei das Polypeptid IL-19-Aktivität aufweist;
(f) einem Polypeptid bestehend aus mindestens 50 Aminosäuren der Aminosäurereste -24 bis 153 von SEQ ID NO:2, wobei das Polypeptid IL-19-Aktivität aufweist;
(g) einem Polypeptid bestehend aus mindestens 30 Aminosäuren des Polypeptids in voller Länge, welches von der cDNA enthalten in ATCC 97662 codiert wird, wobei das Polypeptid IL-19-Aktivität aufweist; und
(h) einem Polypeptid bestehend aus mindestens 50 Aminosäuren des Polypeptids in voller Länge, welches von der cDNA enthalten in ATCC 97662 codiert wird, wobei das Polypeptid IL-19-Aktivität aufweist, wobei die IL-19-Aktivität nach einem von (a) bis (h) die Eigenschaft einer Modulation der Synthese von mindestens einem Cytokin in der Gruppe bestehend aus IFN-γ, Lymphotoxin, IL-2, IL-3 und GM-GSF in einer Population von T-Helferzellen ist, die induziert ist, um eines oder mehrere von diesen Cytokinen zu synthetisieren, indem sie syngenen Antigen-präsentierenden Zellen (APCs) und Antigen ausgesetzt wird.

16. Polypeptid nach Anspruch 14 oder 15, wobei das Polypeptid radioaktiv markiert oder glycosyliert ist.

17. Polypeptid nach einem der Ansprüche 13 bis 15, wobei das Polypeptid an ein heterologes Polypeptid fusioniert ist.

18. Polypeptid nach einem der Ansprüche 14 bis 17, wobei dem Polypeptid ein N-terminales Methionin fehlt.

19. Antikörper, welcher spezifisch an das Polypeptid nach einem der Ansprüche 14 bis 16 bindet.

20. Antikörper nach Anspruch 19, wobei der Antikörper polyclonal oder monoclonal ist.

21. Nucleinsäuremolekül, welches ein Polynucleotid umfasst, welches unter stringenten Hybridisierungsbedingungen an ein Polynucleotid nach einem der Ansprüche 1 bis 3 hybridisiert, wobei der komplementäre Strang des Polynucleotids, welcher hybridisiert, zu mindestens 90% identisch mit einem Polynucleotid nach einem der Ansprüche 1 bis 3 ist.

22. Antagonist des Polypeptids nach Anspruch 14 oder 15, wobei der Antagonist der Antikörper nach Anspruch 19 oder 20 ist.

23. Arzneimittel, welches das Polypeptid nach einem der Ansprüche 14 bis 18, den Antikörper nach Anspruch 19 oder 20 oder den Antagonist nach Anspruch 22 und gegebenenfalls einen pharmazeutisch verträglichen Träger umfasst.

24. Diagnosemittel, welches das Polynucleotid nach einem der Ansprüche 1 bis 3 oder den Antikörper nach Anspruch 19 oder 20 umfasst.

25. Verwendung des Polypeptids nach einem der Ansprüche 14 bis 18 für die Herstellung eines Arzneimittels zur Behandlung eines Individuums, das reduzierter Spiegel an IFN-γ, TNF-α und/oder IL-6 bedarf, von rheumatoider Arthritis, systemischem Lupus erythematodes (SLE), Myasthenia gravis, insulinabhängigem Diabetes mellitus, Thyroiditis, parasitären Infektionen oder zur "Adoptiven Immuntherapie" von Krebs.

26. Verwendung des Antagonisten nach Anspruch 22 oder des Antikörpers nach Anspruch 19 oder 20 für die Herstellung eines Arzneimittels zur Erhöhung der IL-2- Produktion.

27. Verwendung des Polypeptids nach einem der Ansprüche 14 bis 18 in der Herstellung eines Medikaments.

28. Verwendung des Antikörpers nach einem der Ansprüche 19 oder 20 in der Herstellung eines Medikaments.

29. Verwendung des Antagonisten nach Anspruch 22 in der Herstellung eines Medikaments.

30. Arzneimittel zur Behandlung eines Individuums, das reduzierter Spiegel an IFN-γ, TNF-α und/oder IL-6 bedarf, von rheumatoider Arthritis, systemischem Lupus erythematodes (SLE), Myasthenia gravis, insulinabhängigem Diabetes mellitus, Thyroiditis, parasitären Infektionen oder zur "Adoptiven Immunotherapie" von Krebs, umfassend das Polypeptid nach einem der Ansprüche 14 bis 18.

31. Arzneimittel zur Erhöhung der IL-2-Produktion, welches den Antagonisten nach Anspruch 22 oder den Antikörper nach Anspruch 19 oder 20 umfasst.

## Revendications

1. Polynucléotide choisi parmi le groupe constitué par :
(a) un polynucléotide codant pour les résidus 1 à 153 de SEQ ID NO:2;
(b) un polynucléotide codant pour les résidus -24 à 153 de SEQ ID NO:2;
(c) un polynucléotide ayant la séquence de nucléotides 116 à 574 de SEQ ID NO:1 ;
(d) un polynucléotide ayant la séquence de nucléotides 44 à 574 de SEQ ID NO:1;
(e) un polynucléotide codant pour la forme mature du polypeptide codé par l'ADNc contenu dans ATCC 97662 ;
(f) un polynucléotide codant pour le polypeptide de pleine longueur codé par l'ADNc contenu dans ATCC 97662 ;
(g) un polynucléotide ayant la séquence codante de l'ADNc contenu dans ATCC 97662 codant pour la forme mature du polypeptide ;
(h) un polynucléotide ayant la séquence codante de l'ADNc contenu dans ATCC 97662 codant pour le polypeptide de pleine longueur ;
(i) un polynucléotide codant pour une portion porteuse d'épitope immunogène d'un polypeptide codé par un polynucléotide de l'un quelconque des points (a) à (h), dans lequel ledit épitope immunogène est une portion du polypeptide qui déclenche une réponse d'anticorps lorsque le polypeptide complet est l'immunogène ;
(j) un polynucléotide codant pour une portion porteuse d'épitope d'un polypeptide IL-19 comprenant les résidus d'acides aminés VDNHGLRRC ou RVFKDHQEPNPKILRKISS ;
(k) un polynucléotide identique à au moins 90 % au polynucléotide tel que défini dans l'un quelconque des points (a) à (h) et qui code pour un polypeptide ayant une activité d'IL-19, dans lequel ladite activité est la propriété de moduler la synthèse d'au moins une cytokine dans le groupe constitué par IFN-γ, lymphotoxine, IL-2, IL-3 et GM-CSF dans une population de lymphocytes T auxiliaires induite pour synthétiser une ou plusieurs de ces cytokines par exposition à des cellules présentant l'antigène (CPA) syngéniques et à l'antigène ; et
(l) un polynucléotide identique à au moins 90 % au polynucléotide tel que défini dans l'un quelconque des points (a) à (h), dans lequel le brin complémentaire dudit polynucléotide s'hybride dans des conditions d'hybridation stringentes avec le polynucléotide de l'un quelconque des points (a) à (h) ;
ou le brin complémentaire d'un polynucléotide de ce type.

2. Polynucléotide selon la revendication 1, qui est de l'ADN ou de l'ARN.

3. Polynucléotide selon la revendication 2, qui est de l'ADN génomique.

4. Polynucléotide selon l'une quelconque des revendications 1 à 3 fusionné avec un polynucléotide hétérologue.

5. Polynucléotide selon la revendication 4, dans lequel le polynucléotide hétérologue code pour un polypeptide hétérologue.

6. Polynucléotide selon la revendication 5, dans lequel le polypeptide hétérologue est fusionné à un polypeptide codé par le polynucléotide selon l'une quelconque des revendications 1 à 3.

7. Procédé de production d'un vecteur recombinant comprenant l'insertion d'un polynucléotide selon l'une quelconque des revendications 1 à 6 dans un vecteur.

8. Vecteur contenant le polynucléotide selon l'une quelconque des revendications 1 à 6 ou produit par le procédé selon la revendication 7.

9. Vecteur selon la revendication 8, dans lequel le polynucléotide est lié de façon opérationnelle aux séquences de contrôle de l'expression permettant l'expression dans des cellules hôtes procaryotes ou eucaryotes.

10. Procédé de production d'une cellule hôte recombinante comprenant l'introduction du vecteur selon la revendication 8 ou 9 dans une cellule hôte.

11. Cellule hôte génétiquement modifiée avec le polynucléotide selon l'une quelconque des revendications 1 à 6 ou le vecteur selon la revendication 8 ou 9 ou produite par le procédé selon la revendication 10.

12. Cellule hôte selon la revendication 11, dans laquelle ladite cellule est une cellule procaryote, une cellule eucaryote, une cellule animale, une cellule de mammifère, une cellule d'insecte, une cellule végétale, une cellule fongique, une cellule COS, une cellule CHO, ou une cellule d'*E*. *coli.*

13. Procédé de production d'un polypeptide ayant une activité d'IL-19, dans lequel ladite activité est la propriété de moduler la synthèse d'au moins une cytokine dans le groupe constitué par IFN-γ, lymphotoxine, IL-2, IL-3 et GM-CSF dans une population de lymphocytes T auxiliaires induite pour synthétiser une ou plusieurs de ces cytokines par exposition à des cellules présentant l'antigène (CPA) syngéniques et à l'antigène, comprenant : la culture de la cellule hôte selon la revendication 11 ou 12 dans des conditions telles que ledit polypeptide est exprimé, et la récupération du polypeptide codé par ledit polynucléotide à partir de la culture.

14. Polypeptide ayant la séquence d'acides aminés codée par un polynucléotide selon l'une quelconque des revendications 1 à 6, ou pouvant être obtenu par le procédé selon la revendication 13.

15. Polypeptide choisi parmi le groupe constitué par :
(a) un polypeptide ayant une séquence d'acides aminés identique à au moins 80 % aux résidus d'acides aminés -24 à 153 de SEQ ID NO:2, dans lequel ledit polypeptide a une activité d'IL-19 ;
(b) un polypeptide ayant une séquence d'acides aminés identique à au moins 80 % aux résidus d'acides aminés 1 à 153 de SEQ ID NO:2, dans lequel ledit polypeptide a une activité d'IL-19 ;
(c) un polypeptide ayant une séquence d'acides aminés identique à au moins 80 % au polypeptide de pleine longueur codé par l'ADNc contenu dans ATCC 97662, dans lequel ledit polypeptide a une activité d'IL-19 ;
(d) un polypeptide ayant une séquence d'acides aminés identique à au moins 80% à la forme mature du polypeptide codé par l'ADNc contenu dans ATCC 97662, dans lequel ledit polypeptide a une activité d'IL-19 ;
(e) un polypeptide constitué d'au moins 30 acides aminés contigus des résidus d'acides aminés -24 à 153 de SEQ ID NO:2, dans lequel ledit polypeptide a une activité d'IL-19 ;
(f) un polypeptide constitué d'au moins 50 acides aminés des résidus d'acides aminés -24 à 153 de SEQ ID NO:2, dans lequel ledit polypeptide a une activité d'IL-19 ;
(g) un polypeptide constitué d'au moins 30 acides aminés du polypeptide de pleine longueur codé par l'ADNc contenu dans ATCC 97662, dans lequel ledit polypeptide a une activité d'IL-19 ; et
(h) un polypeptide constitué d'au moins 50 acides aminés du polypeptide de pleine longueur codé par l'ADNc contenu dans ATCC 97662, dans lequel ledit polypeptide a une activité d'IL-19, dans laquelle ladite activité d'IL-19 de l'un quelconque des points (a) à (h) est la propriété de moduler la synthèse d'au moins une cytokine dans le groupe constitué par IFN-γ, lymphotoxine, IL-2, IL-3 et GM-CSF dans une population de lymphocytes T auxiliaires induite pour synthétiser une ou plusieurs de ces cytokines par exposition à des cellules présentant l'antigène (CPA) syngéniques et à l'antigène.

16. Polypeptide selon l'une ou l'autre des revendications 14 ou 15, dans lequel ledit polypeptide est radio-marqué ou glycosylé.

17. Polypeptide selon l'une quelconque des revendications 13 à 15, dans lequel ledit polypeptide est fusionné avec un polypeptide hétérologue.

18. Polypeptide selon l'une quelconque des revendications 14 à 17, dans lequel ledit polypeptide est dépourvu d'une méthionine N-terminale.

19. Anticorps qui se lie spécifiquement au polypeptide selon l'une quelconque des revendications 14 à 16.

20. Anticorps selon la revendication 19, dans lequel ledit anticorps est polyclonal ou monoclonal.

21. Molécule d'acide nucléique comprenant un polynucléotide, qui s'hybride dans des conditions d'hybridation stringentes à un polynucléotide selon l'une quelconque des revendications 1 à 3, dans laquelle le brin complémentaire dudit polynucléotide qui s'hybride est identique à au moins 90 % à un polynucléotide selon l'une quelconque des revendications 1 à 3.

22. Antagoniste du polypeptide selon l'une ou l'autre des revendications 14
ou 15, dans lequel ledit antagoniste est l'anticorps selon l'une ou l'autre des revendications 19 et 20.

23. Composition pharmaceutique comprenant le polypeptide selon l'une quelconque des revendications 14 à 18, l'anticorps selon l'une ou l'autre des revendications 19 ou 20, ou l'antagoniste selon la revendication 22, et éventuellement un support pharmaceutiquement acceptable.

24. Composition de diagnostic comprenant le polynucléotide selon l'une quelconque des revendications 1 à 3 ou l'anticorps selon l'une ou l'autre des revendications 19 ou 20.

25. Utilisation du polypeptide selon l'une quelconque des revendications 14 à 18 pour la préparation d'une composition pharmaceutique pour le traitement d'un individu ayant besoin de niveaux diminués d'IFN-γ, de TNF-α, et/ou d'IL-6, de la polyarthrite rhumatoïde, du lupus érythémateux systémique (LES), de la myasthénie gravis, du diabète insulinodépendant, de la thyroïdite, des infections parasitiques ou pour « l'immunothérapie adoptive » du cancer.

26. Utilisation de l'antagoniste selon la revendication 22 ou de l'anticorps selon la revendication 19 ou 20 pour la préparation d'une composition pharmaceutique pour augmenter la production d'IL-2.

27. Utilisation du polypeptide selon l'une quelconque des revendications 14 à 18 dans la préparation d'un médicament.

28. Utilisation de l'anticorps selon l'une quelconque des revendications 19
ou 20 dans la préparation d'un médicament.

29. Utilisation de l'antagoniste selon la revendication 22 dans la préparation d'un médicament.

30. Composition pharmaceutique pour le traitement d'un individu ayant besoin de niveaux diminués d'IFN-γ, de TNF-α, et/ou d'IL-6, de la polyarthrite rhumatoïde, du lupus érythémateux systémique (LES), de la myasthénie gravis, du diabète insulinodépendant, de la thyroïdite, des infections parasitiques ou pour « l'immunothérapie adoptive » du cancer, comprenant le polypeptide selon l'une quelconque des revendications 14 à 18.

31. Composition pharmaceutique pour augmenter la production d'IL-2, comprenant l'antagoniste de la revendication 22 ou l'anticorps de la revendication 19
ou 20.
